# EUROPÄISCHE PATENTSCHRIFT

(11) **EP 2 734 179 B1**
(45) Veröffentlichungstag und Bekanntmachung des Hinweises auf die Patenterteilung: **01.03.2017**
(21) Anmeldenummer: 12724614.8
(22) Anmeldetag: 29.05.2012
(51) Int. Cl.: A61K 8/41, A61K 8/49, A61Q 5/10, C07C 211/63, C07D 295/13

(54) **NEUE OXIDATIONSFARBSTOFFVORPRODUKTE**
NOVEL OXIDATION DYE PRECURSORS
NOUVEAUX PRÉCURSEURS DE COLORANT D'OXYDATION

(30) Priorität: 22.07.2011 DE 102011079643
(43) Veröffentlichungstag der Anmeldung: 28.05.2014
(73) Patentinhaber: Henkel AG & Co. KGaA, 40589 Düsseldorf (DE)
(72) Erfinder: GROß, Wibke, 41836 Hückelhoven (DE); NEMITZ, Ralph, 41363 Jüchen (DE); KROOS, Astrid, 40789 Monheim (DE)
(86) Internationale Anmeldenummer: PCT/EP2012/060035
(87) Internationale Veröffentlichungsnummer: WO 2013/013861

(56) Entgegenhaltungen:
- EP-A1- 1 396 486
- EP-A1- 1 739 079
- EP-A1- 1 739 084
- EP-A2- 1 020 176
- FR-A1- 2 776 290

## Beschreibung

Die Erfindung betrifft ein Mittel zur oxidativen Farbveränderung von keratinhaltigen Fasern, insbesondere menschlichen Haaren, welches bestimmte kationische, dimere p-Phenylendiaminderivate enthält, die Verwendung dieser Mittel als Farbveränderungsmittel von keratinhaltigen Fasern zur Verbesserung der Färbeergebnisse sowie ein Verfahren zum Färben von keratinhaltigen Fasern, insbesondere menschlichen Haaren.

Die Veränderung von Form und Farbe der Haare stellt einen wichtigen Bereich der modernen Kosmetik dar. Zur modischen Farbgestaltung von Frisuren oder zur Kaschierung von ergrautem oder gar weißem Haar mit modischen oder natürlichen Farbtönen greift der Verbraucher zu farbverändernden Mitteln.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung bzw. Farbveränderung diverse Systeme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch intensive, hervorragende, lang anhaltende Färbeergebnisse aus. Für natürlich wirkende Färbungen kann eine Mischung aus einer größeren Zahl von Oxidationsfarbstoffvorprodukten eingesetzt werden, wobei in vielen Fällen weiterhin zusätzlich direktziehende Farbstoffe zur Nuancierung verwendet werden.

Insbesondere oxidative Haarfärbemittel sind trotz ihrer vorteilhaften Färbeeigenschaften für den Anwender mit Nachteilen behaftet. Insbesondere wird für einige der gängigen Oxidationsfarbstoffvorprodukte, darunter p-Phenylendiamin, vermutet, für manche Verbraucher irritierend oder reizend zu wirken und dadurch Sensibilisierungen oder gar allergische Reaktionen auszulösen. Daher besteht für diese Substanzen noch weiterhin Verbesserungsbedarf in ihrem physiologischen Verträglichkeitsprofil. Auf der Suche nach Ersatzstoffen wurden viele Verbindungen erforscht, die aber häufig unter anwendungstechnischen Problemen, insbesondere mangelndem Grauabdeckungsvermögen, leiden. Außerdem besteht trotz bereits hoch entwickelter Färbesysteme weiterhin Bedarf an Färbesystemen, die hervorragende Leuchtkraft und Intensität der Färbungen erreichen, gleichzeitig jedoch über eine sehr gute Haltbarkeit und eine hervorragende Homogenität verfügen.

Aufgabe der vorliegenden Erfindung ist es daher, die oben genannten Nachteile oxidativer Haarfärbemittel herabzusenken. Die Färbemittel sollen intensive Färbungen mit hoher Farbigkeit und mit einer guten Beständigkeit gegenüber äußeren Einflüssen, insbesondere mit guter Lichtechtheit und Waschechtheit, erzeugen, welche auch nach mehrmaligem Shampoonieren der Haare keine Farbabschwächung oder Farbverschiebung erleiden. Darüber hinaus sollen die Färbungen möglichst ein hervorragendes Egalisiervermögen aufweisen und wenig selektiv sein, d.h. auf unterschiedlich vorbehandeltem Haar möglichst gleichmäßige, einheitliche Färbeergebnisse erzielen. Außerdem sollen die Färbemittel ein toxikologisch vorteilhaftes Profil besitzen.

Es wurde gefunden, dass sich bestimmte dimere p-Phenylendiaminderivate mit kationischer Ladung als Oxidationsfarbstoffvorprodukte hervorragend zum Färben von keratinhaltigen Fasern eignen. Sie ergeben Ausfärbungen mit hoher Farbintensität und hervorragender Brillanz sowie exzellenter Grauabdeckung.

Aus FR 2864964 sind kationische, dimere direktziehende Azofarbstoffe für die Haarfärbung bekannt. Dimere, ungeladene dimere p-Phenylendiamine als Entwicklerkomponenten für oxidative Haarfärbemittel sind aus EP 1739084 bzw. EP 1739079 bekannt.

EP 1396486 offenbart dikationische bis-Paraphenylendiamin-Derivate als Entwicklerkomponenten, die am aromatischen Ring jeweils mit einer Pyrrolidingruppe substituiert sind.

Dimere p-Phenylendiaminderivate mit kationischer Ladung als Oxidationsfarbstoffvorprodukte entsprechend nachfolgender Formel (I) sind bislang nicht bekannt.

Ein erster Gegenstand der Erfindung ist daher ein Mittel zur oxidativen Farbveränderung keratinischer Fasern, welches dadurch gekennzeichnet ist, dass es in einem kosmetischen Träger als Oxidationsfarbstoffvorprodukt vom Entwickler-Typ mindestens eine Verbindung der Formel (I) 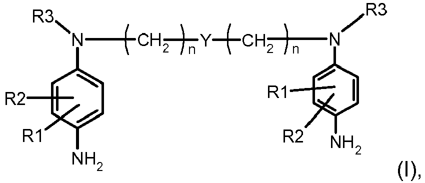 in der
- R1 und R2: unabhängig voneinander für Wasserstoff, eine C₁-C₆-Alkylgruppe, eine C₂-C₆-Alkenylgruppe, eine C₁-C₆-Hydroxyalkylgruppe, eine C₂-C₆-Polyhydroxy-alkylgruppe, eine C₁-C₆-Alkoxygruppe oder ein Halogenatom stehen,
- R3: für Wasserstoff, eine C₁-C₆-Alkylgruppe, eine C₂-C₆-Alkenylgruppe, eine C₂-C₆-Hydroxyalkylgruppe, eine C₂-C₆-Polyhydroxyalkylgruppe, eine C₁-C₆-Alkoxy-C₂-C₆-alkylgruppe, eine Cyano-C₁-C₆-alkylgruppe oder eine Aryl-C₁-C₆-alkylgruppe steht,
- n: für eine ganze Zahl von 2 bis 6 steht,
- Y: für eine kationische Gruppierung der Formeln (II) bis (V) steht, 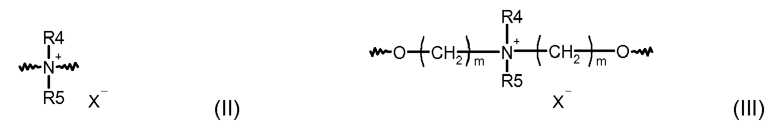 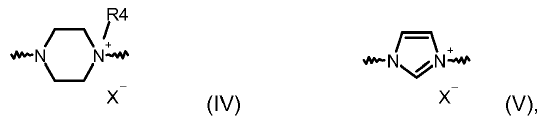
worin
- R4, R5: unabhängig voneinander für eine C₁-C₆-Alkylgruppe, eine C₂-C₆-Alkenylgruppe, eine C₂-C₆-Hydroxyalkylgruppe, eine C₂-C₆-Polyhydroxyalkylgruppe, eine C₁-C₆-Alkoxy-C₂-C₆-alkylgruppe, eine Cyano-C₁-C₆-alkylgruppe oder eine Aryl-C₁-C₆-alkylgruppe stehen,
- m: für eine ganze Zahl von 2 bis 6 steht und
- X⁻: für ein physiologisch verträgliches Anion steht,
und/oder deren physiologisch verträgliches Salz enthält.

Unter keratinischen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

Die erfindungsgemäßen Mittel enthalten die Verbindungen der Formel (I) in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die Farbstoffvorprodukte gemäß Formel (I) in eine pulverförmige oder auch tabletten-förmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Im Folgenden werden Beispiele für die in Formel (I) genannten Substituenten R1, R2 sowie R3, R4, R5 und R6 exemplarisch genannt:
Beispiele für C₁-C₆-Alkylreste sind -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, -(CH₂)₄CH₃, -(CH₂)₅CH₃. Besonders bevorzugte Alkylreste sind Methyl und Ethyl. Beispiele für C₂-C₆-Alkenylgruppen sind Prop-2-enyl (Allylgruppe), 2-Methyl-prop-2-enyl, But-3-enyl, But-2-enyl, Pent-4-enyl oder Pent-3-enyl. Beispiele für C₁-C₆-Hydroxyalkylgruppen sind -CH₂OH, -CH₂CH₂OH, -CH₂CH₂CH₂OH, -CH₂CH(OH)CH₃, -CH₂CH₂CH₂CH₂OH, wobei -CH₂CH₂OH bevorzugt ist. Beispiele für C₂-C₆-Polyhydroxyalkylgruppen sind 2,3-Dihydroxypropylgruppe, 3,4-Dihydroxybutylgruppe, 2,4-Dihydroxybutylgruppe und 1,2-Dihydroxyethylgruppe. Beispiele für C₁-C₆-Alkoxygruppen sind -OCH₃, -OCH₂CH₃, -OCH₂CH₂CH₃, -OCH(CH₃)₂ oder -OC(CH₃)₃, bevorzugt ist die Methoxygruppe (-OCH₃). Beispiele für C₁-C₆-Alkoxy-C₂-C₆-alkylgruppen sind -CH₂OCH₃, -CH₂OCH₂CH₃, -CH₂OCH(CH₃)₂, -CH₂CH₂OCH₃, -CH₂CH₂CH₂OCH₃, -CH₂CH₂OCH₂CH₃, -CH₂CH₂CH₂OCH₂CH₃, -CH₂CH₂OCH(CH₃)₂, -CH₂CH₂CH₂OCH(CH₃)₂. Beispiele für Halogen sind Fluor, Chlor, Brom oder Iod, insbesondere Fluor und Chlor. Beispiele für Cyano-C₁-C₆-alkylgruppen sind -CH₂CN, -C₂H₄CN und -C₃H₆CN. Beispiele für Aryl-C₁-C₆-alkylgruppen sind Benzyl, 1-Phenylethyl und 2-Phenylethyl.

In einer Ausführungsform des ersten Erfindungsgegenstands ist ein erfindungsgemäßes Mittel dadurch gekennzeichnet, dass es mindestens eine Verbindung der Formel (I) enthält, in der R1 und R2 unabhängig voneinander für Wasserstoff, eine C₁-C₆-Alkylgruppe, eine C₁-C₆-Alkoxygruppe oder ein Halogenatom stehen.

Eine weitere Ausführungsform des ersten Erfindungsgegenstands ist daher dadurch gekennzeichnet, dass es mindestens eine Verbindung der Formel (I) enthält, in der R3 für Wasserstoff oder eine C₁-C₆-Alkylgruppe, insbesondere für Wasserstoff, steht.

Besonders bevorzugt steht n für die Zahl 2 oder 3.

Eine weitere Ausführungsform des ersten Erfindungsgegenstands ist daher dadurch gekennzeichnet, dass das Mittel mindestens eine Verbindung der Formel (I) enthält, in der R4 und R5 unabhängig voneinander für eine C₁-C₆-Alkylgruppe oder eine C₂-C₆-Alkenylgruppe stehen und/oder m bevorzugt für die Zahl 2 oder 3 steht.

Besonders vorteilhafte Effekte werden erzielt, wenn Y für eine kationische Gruppierung der Formeln (II) oder (IV) steht.

Eine weitere Ausführungsform des ersten Erfindungsgegenstands ist daher dadurch gekennzeichnet, dass das Mittel mindestens eine Verbindung der Formel (I) enthält, in der Y für eine kationische Gruppierung der Formeln (II) oder (IV) steht.

Besonders vorteilhafte Verbindungen werden erhalten, wenn R1 und R2 unabhängig voneinander für Wasserstoff, eine C₁-C₆-Alkylgruppe, eine C₁-C₆-Alkoxygruppe oder ein Halogenatom stehen, R3 für Wasserstoff steht, n für 2 oder 3 steht, Y für eine kationische Gruppierung der Formeln (II) oder (IV) steht und R4 und R5 unabhängig voneinander für eine C₁-C₆-Alkylgruppe oder eine C₂-C₆-Alkenylgruppe stehen.

X⁻ ist ein physiologisch verträgliches Anion. X⁻ ist daher bevorzugt ausgewählt aus Chlorid, ½ Sulfat, Hydrogensulfat, Bromid, Benzolsulfonat, p-Toluolsulfonsulfonat, C₁-C₄-Alkansulfonat, C₁-C₄-Alkansulfat oder Trifluormethansulfonat sowie Acetat, Lactat, Citrat, Tartrat und/oder den anionischen Salzanteilen weiterer physiologisch verträglicher, organischer Säuren.

Eine weitere Ausführungsform des ersten Erfindungsgegenstands ist dadurch gekennzeichnet, dass das Mittel mindestens eine Verbindung gemäß Formel (I) enthält, die ausgewählt ist aus Salzen
des N,N-Dimethyl-3-[(4-aminophenyl)amino]-N-{3-[(4-aminophenyl)amino]propyl}-1-propanaminiums 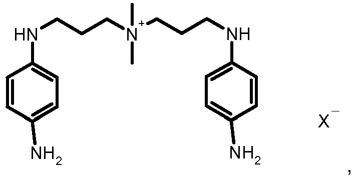 des N-Ethyl-N-methyl-3-[(4-aminophenyl)amino]-N-{3-[(4-aminophenyl)amino]propyl}-1-propanaminiums 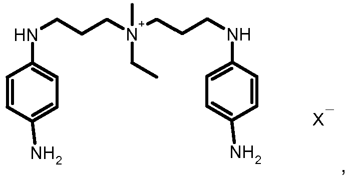 des N-Methyl-N-propyl-3-[(4-aminophenyl)amino]-N-{3-[(4-aminophenyl)amino]propyl}-1-propanaminiums 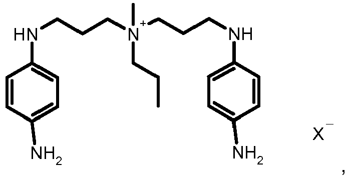 des N-Allyl-N-methyl-3-[(4-aminophenyl)amino]-N-{3-[(4-aminophenyl)amino]-propyl}-1-propanaminiums 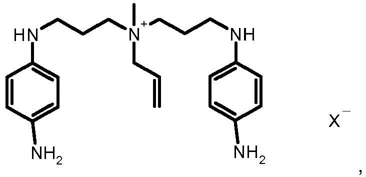 des N,N-Dimethyl-2-[(4-aminophenyl)amino]-N-{2-[(4-aminophenyl)amino]ethyl}-1-ethanaminiums 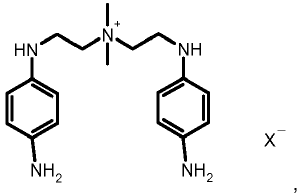 des N-Ethyl-N-methyl-2-[(4-aminophenyl)amino]-N-{2-[(4-aminophenyl)amino]ethyl}-1-ethanaminiums 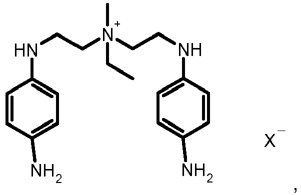 des N-Methyl-N-propyl-2-[(4-aminophenyl)amino]-N-{2-[(4-aminophenyl)amino]ethyl}-1-ethanaminiums 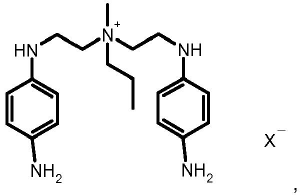 des N-Allyl-N-methyl-2-[(4-aminophenyl)amino]-N-{2-[(4-aminophenyl)amino]ethyl}-1-ethanaminiums 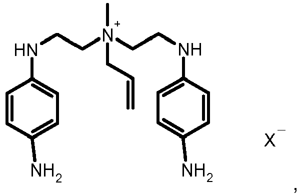 des N1,N4-Bis-{3-[(4-aminophenyl)amino]propyl}-N1-methylpiperaziniums 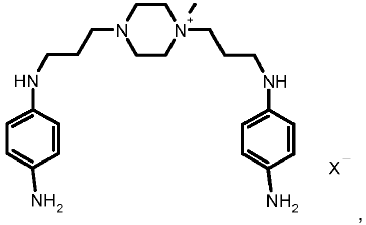 des N1,N4-Bis-{3-[(4-aminophenyl)amino]propyl}-N1-ethylpiperaziniums 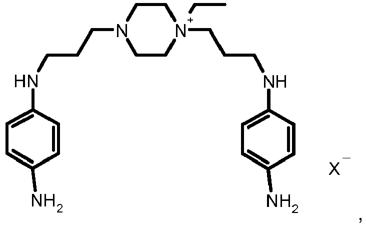 des N1,N4-Bis-{3-[(4-aminophenyl)amino]propyl}-N1-propylpiperaziniums 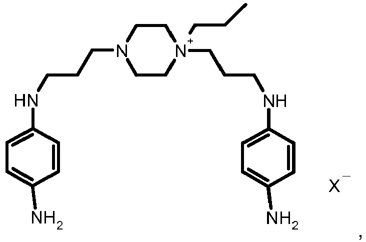 des N1,N4-Bis-{3-[(4-aminophenyl)amino]propyl}-N1-allylpiperaziniums 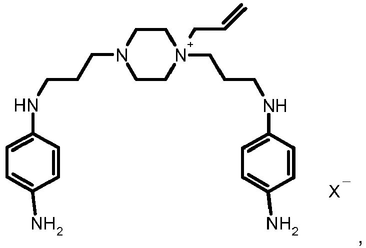 des N1,N4-Bis-{2-[(4-aminophenyl)amino]ethyl}-N1-methylpiperaziniums 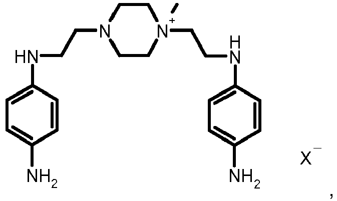 des N1,N4-Bis-{2-[(4-aminophenyl)amino]ethyl}-N1-ethylpiperaziniums 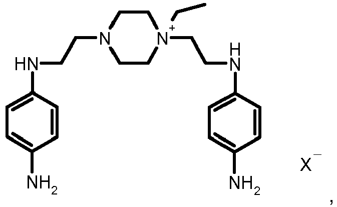 des N1,N4-Bis-{2-[(4-aminophenyl)amino]ethyl}-N1-propylpiperaziniums 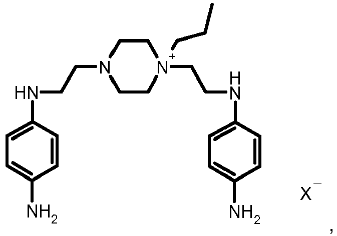 des N1,N4-Bis-{2-[(4-aminophenyl)amino]ethyl}-N1-allylpiperaziniums 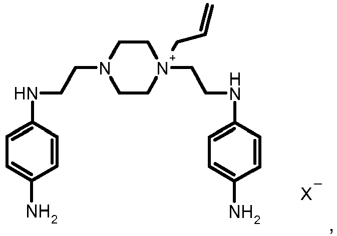 des N,N-Dimethyl-3-[(4-amino-3-methylphenyl)amino]-N-{3-[(4-amino-3-methylphenyl)amino]-propyl}-1-propanaminiums 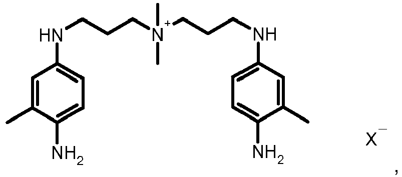 des N-Ethyl-N-methyl-3-[(4-amino-3-methylphenyl)amino]-N-{3-[(4-amino-3-methylphenyl)amino]-propyl}-1-propanaminiums 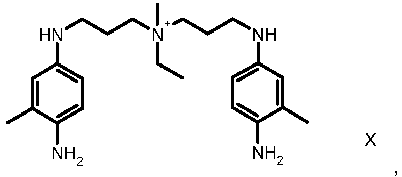 des N-Methyl-N-propyl-3-[(4-amino-3-methylphenyl)amino]-N-{3-[(4-amino-3-methylphenyl)amino]propyl}-1-propanaminiums 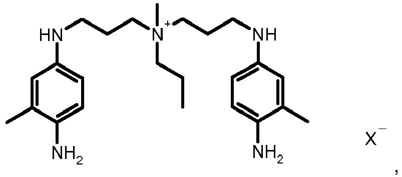 des N-Allyl-N-methyl-3-[(4-amino-3-methylphenyl)amino]-N-{3-[(4-amino-3-methylphenyl)amino]-propyl}-1-propanaminiums 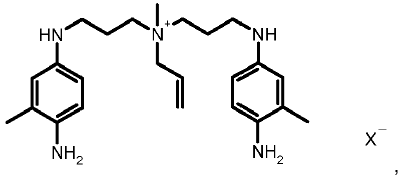 des N,N-Dimethyl-2-[(4-amino-3-methylphenyl)amino]-N-{2-[(4-amino-3-methylphenyl)amino]-ethyl}-1-ethanaminiums 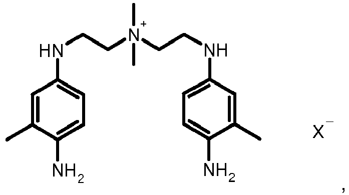 des N-Ethyl-N-methyl-2-[(4-amino-3-methylphenyl)amino]-N-{2-[(4-amino-3-methylphenyl)amino]-ethyl}-1-ethanaminiums 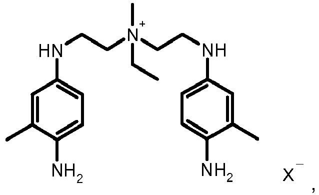 des N-Methyl-N-propyl-2-[(4-amino-3-methylphenyl)amino]-N-{2-[(4-amino-3-methylphenyl)-amino]ethyl}-1-ethanaminiums 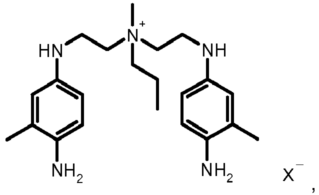 des N-Allyl-N-methyl-2-[(4-amino-3-methylphenyl)amino]-N-{2-[(4-amino-3-methylphenyl)amino]-ethyl}-1-ethanaminiums 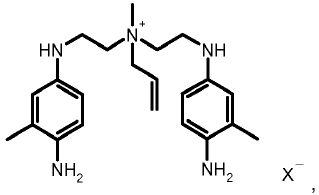 des N,N-Dimethyl-3-[(4-amino-3-methoxyphenyl)amino]-N-{3-[(4-amino-3-methoxyphenyl)amino]-propyl}-1-propanaminiums 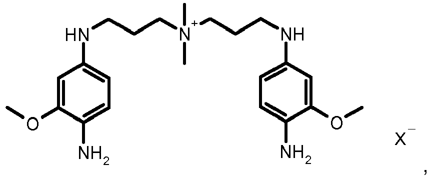 des N-Ethyl-N-methyl-3-[(4-amino-3-methoxyphenyl)amino]-N-{3-[(4-amino-3-methoxyphenyl)amino]propyl}-1-propanaminiums 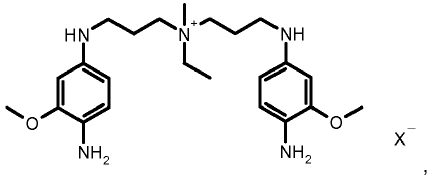 des N-Methyl-N-propyl-3-[(4-amino-3-methoxyphenyl)amino]-N-{3-[(4-amino-3-methoxyphenyl)-amino]propyl}-1-propanaminiums 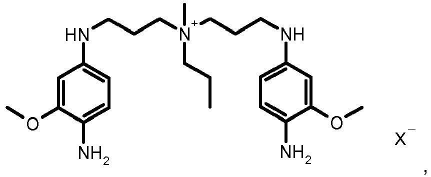 des N-Allyl-N-methyl-3-[(4-amino-3-methoxyphenyl)amino]-N-{3-[(4-amino-3-methoxyphenyl)-amino]propyl}-1-propanaminiums 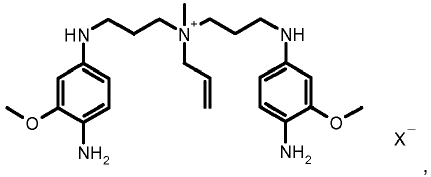 des N,N-Dimethyl-2-[(4-amino-3-methoxyphenyl)amino]-N-{2-[(4-amino-3-methoxyphenyl)-amino]ethyl}-1-ethanaminiums 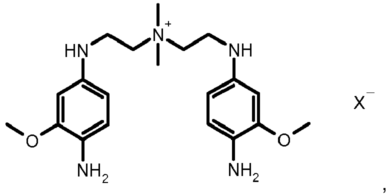 des N-Ethyl-N-methyl-2-[(4-amino-3-methoxyphenyl)amino]-N-{2-[(4-amino-3-methoxyphenyl)-amino]ethyl}-1-ethanaminiums 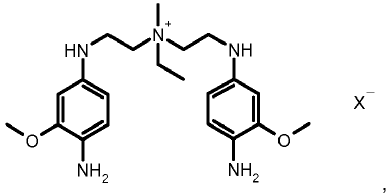 des N-Methyl-N-propyl-2-[(4-amino-3-methoxyphenyl)amino]-N-{2-[(4-amino-3-methoxyphenyl)-amino]ethyl}-1-ethanaminiums 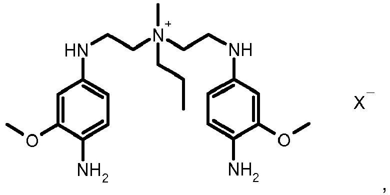 des N-Allyl-N-methyl-2-[(4-amino-3-methoxyphenyl)amino]-N-{2-[(4-amino-3-methoxyphenyl)-amino]ethyl}-1-ethanaminiums 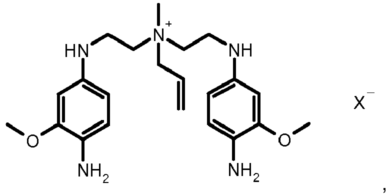 des N,N-Dimethyl-3-[(4-amino-3-chlorphenyl)amino]-N-{3-[(4-amino-3-chlorphenyl)amino]propyl}-1-propanaminiums 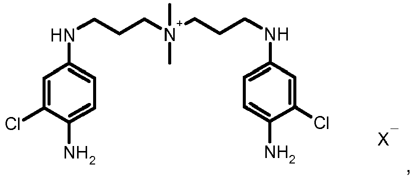 des N-Ethyl-N-methyl-3-[(4-amino-3-chlorphenyl)amino]-N-{3-[(4-amino-3-chlorphenyl)amino]-propyl}-1-propanaminiums 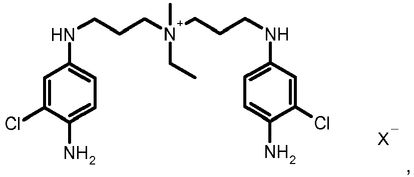 des N-Methyl-N-propyl-3-[(4-amino-3-chlorphenyl)amino]-N-{3-[(4-amino-3-chlorphenyl)amino]-propyl}-1-propanaminiums 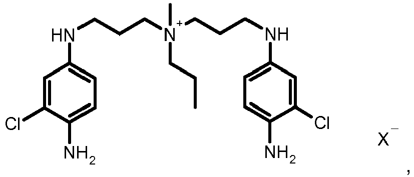 des N-Allyl-N-methyl-3-[(4-amino-3-chlorphenyl)amino]-N-{3-[(4-amino-3-chlorphenyl)amino]-propyl}-1-propanaminiums 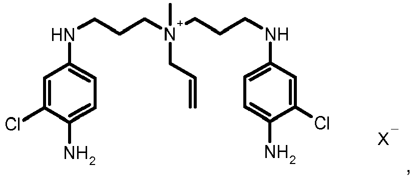 des N,N-Dimethyl-2-[(4-amino-3-chlorphenyl)amino]-N-{2-[(4-amino-3-chlorphenyl)amino]ethyl}-1-ethanaminiums 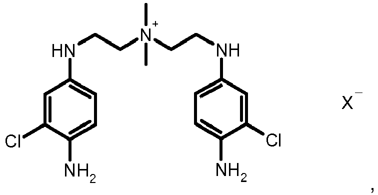 des N-Ethyl-N-methyl-2-[(4-amino-3-chlorphenyl)amino]-N-{2-[(4-amino-3-chlorphenyl)amino]-ethyl}-1-ethanaminiums 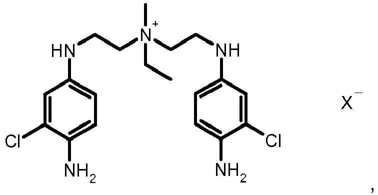 des N-Methyl-N-propyl-2-[(4-amino-3-chlorphenyl)amino]-N-{2-[(4-amino-3-chlorphenyl)amino]-ethyl}-1-ethanaminiums 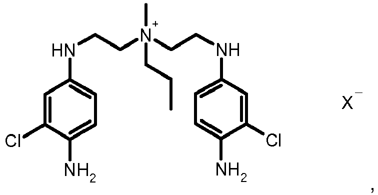 Salze des N-Allyl-N-methyl-2-[(4-amino-3-chlorphenyl)amino]-N-{2-[(4-amino-3-chlorphenyl)amino]ethyl}-1-ethanaminiums 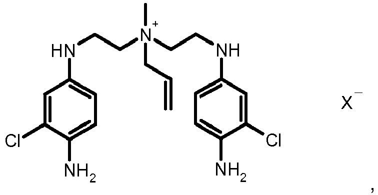 des N,N-Dimethyl-3-[(4-amino-2-methylphenyl)amino]-N-{3-[(4-amino-2-methylphenyl)amino]-propyl}-1-propanaminiums 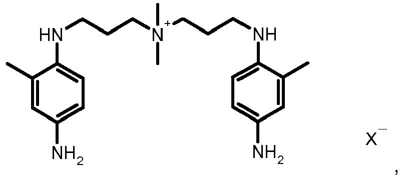 des N-Ethyl-N-methyl-3-[(4-amino-2-methylphenyl)amino]-N-{3-[(4-amino-2-methylphenyl)amino]-propyl}-1-propanaminiums 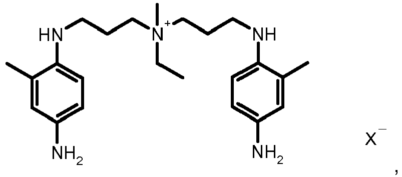 des N-Methyl-N-propyl-3-[(4-amino-2-methylphenyl)amino]-N-{3-[(4-amino-2-methylphenyl)amino]propyl}-1-propanaminiums 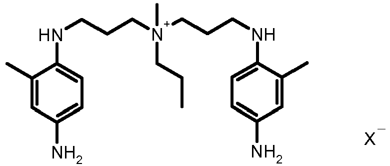 des N-Allyl-N-methyl-3-[(4-amino-2-methylphenyl)amino]-N-{3-[(4-amino-2-methylphenyl)amino]-propyl}-1-propanaminiums 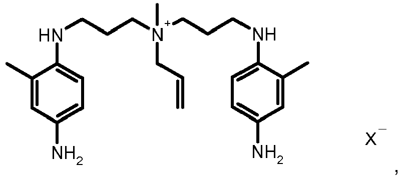 des N1,N4-Bis-{3-[(4-amino-3-methylphenyl)amino]propyl}-N1-methylpiperaziniums 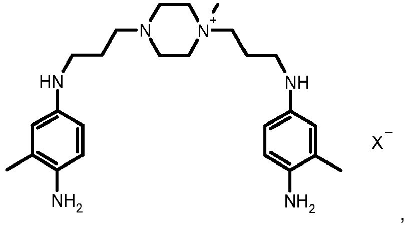 des N1,N4-Bis-{3-[(4-amino-3-methylphenyl)amino]propyl}-N1-ethylpiperaziniums 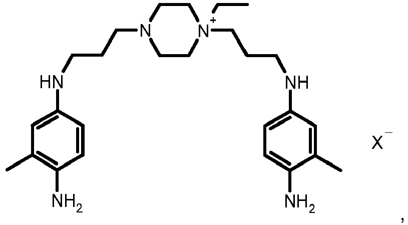 des N1,N4-Bis-{3-[(4-amino-3-methylphenyl)amino]propyl}-N1-propylpiperaziniums 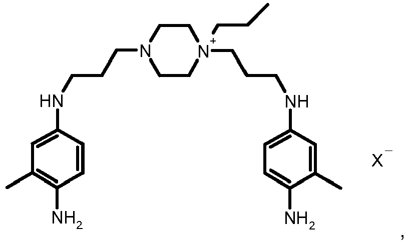 des N1,N4-Bis-{3-[(4-amino-3-methylphenyl)amino]propyl}-N1-allylpiperaziniums 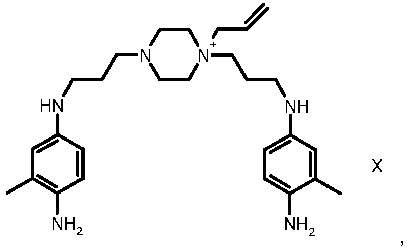 des N1,N4-Bis-{2-[(4-amino-3-methylphenyl)amino]ethyl}-N1-methylpiperaziniums 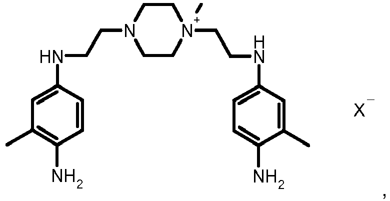 des N1,N4-Bis-{2-[(4-amino-3-methylphenyl)amino]ethyl}-N1-ethylpiperaziniums 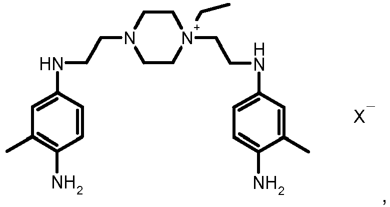 des N1,N4-Bis-{2-[(4-amino-3-methylphenyl)amino]ethyl}-N1-propylpiperaziniums 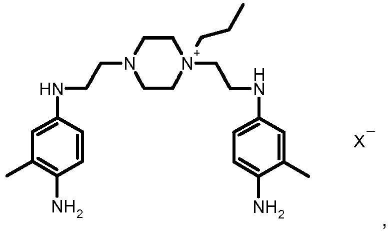 des N1,N4-Bis-{2-[(4-amino-3-methylphenyl)amino]ethyl}-N1-allylpiperaziniums

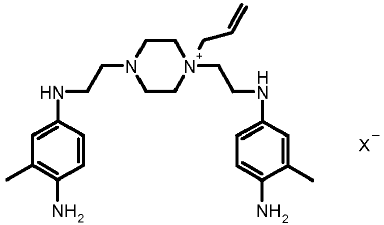

Eine weitere Ausführungsform des ersten Erfindungsgegenstands ist dadurch gekennzeichnet, dass das Mittel mindestens eine Verbindung gemäß Formel (I) enthält, die ausgewählt ist aus Salzen
- des N,N-Dimethyl-3-[(4-aminophenyl)amino]-N-{3-[(4-aminophenyl)amino]propyl}-1-propanaminiums
- des N-Ethyl-N-methyl-3-[(4-aminophenyl)amino]-N-{3-[(4-aminophenyl)amino]propyl}-1-propanaminiums
- des N-Methyl-N-propyl-3-[(4-aminophenyl)amino]-N-{3-[(4-aminophenyl)amino]-propyl}-1-propanaminiums
- des N-Allyl-N-methyl-3-[(4-aminophenyl)amino]-N-{3-[(4-aminophenyl)amino]-propyl}-1-propanaminiums
- des N1,N4-Bis-{3-[(4-aminophenyl)amino]propyl}-N1-methylpiperaziniums
- des N1,N4-Bis-{2-[(4-aminophenyl)amino]ethyl}-N1-methylpiperaziniums
- des N,N-Dimethyl-3-[(4-amino-3-methylphenyl)amino]-N-{3-[(4-amino-3-methylphenyl)amino]-propyl}-1-propanaminiums
- des N-Ethyl-N-methyl-3-[(4-amino-3-methylphenyl)amino]-N-{3-[(4-amino-3-methylphenyl)-amino]propyl}-1-propanaminiums
- des N-Methyl-N-propyl-3-[(4-amino-3-methylphenyl)amino]-N-{3-[(4-amino-3-methylphenyl)-amino]propyl}-1-propanaminiums
- des N,N-Dimethyl-3-[(4-amino-2-methylphenyl)amino]-N-{3-[(4-amino-2-methylphenyl)amino]-propyl}-1-propanaminiums
- des N-Ethyl-N-methyl-3-[(4-amino-2-methylphenyl)amino]-N-{3-[(4-amino-2-methylphenyl)-amino]propyl}-1-propanaminiums
- des N-Methyl-N-propyl-3-[(4-amino-2-methylphenyl)amino]-N-{3-[(4-amino-2-methylphenyl)-amino]propyl}-1-propanaminiums
- des N1,N4-Bis-{3-[(4-amino-3-methylphenyl)amino]propyl}-N1-methylpiperaziniums
- des N1,N4-Bis-{2-[(4-amino-3-methylphenyl)amino]ethyl}-N1-methylpiperaziniums.

Die Verbindungen der Formel (I) können in Form ihrer physiologisch verträglichen Salze, insbesondere der Chloride, der Sulfate und Bromide, eingesetzt werden. Weitere bevorzugte Salze sind von Sulfonsäuren abgeleitet, wie Benzolsulfonate, p-Toluolsulfonsulfonate, C₁-C₄-Alkansulfonate oder Trifluormethansulfonate. Abhängig von der Anzahl der in den erfindungsgemäßen Verbindungen enthaltenen Aminogruppen können Mono-, Di-, Tri-, Tetra- und höhere Addukte als Salze vorliegen.

Erfindungsgemäß bevorzugte Mittel sind dadurch gekennzeichnet, dass sie die Verbindungen gemäß der Formel (I) und/oder deren physiologisch verträglichen Salze, in einem Gewichtsanteil von 0,001 bis 10,0 Gew.-%, insbesondere 0,05 bis 5 Gew.-%, bezogen auf Gesamtgewicht des anwendungsbereiten Mittels, enthalten.

Die Verbindungen der Formel (I) können als alleinige, farbverändernde Verbindungen im erfindungsgemäßen Mittel enthalten sein. Es ist erfindungsgemäß jedoch bevorzugt, wenn das Mittel zusätzlich mindestens ein Oxidationsfarbstoffvorprodukt vom Typ einer Kupplerkomponente enthält.

Kupplerkomponenten bilden im Rahmen der oxidativen Färbung allein keine signifikante Färbung aus, sondern benötigen stets die Gegenwart von Entwicklerkomponenten.

Kupplerkomponenten im Sinne der Erfindung erlauben mindestens eine Substitution eines chemischen Restes des Kupplers durch die oxidierte Form der Entwicklerkomponente. Dabei bilden sich kovalenten Bindungen zwischen Kuppler- und Entwicklerkomponente aus.

Als erfindungsgemäß geeignete Kupplerkomponenten wird bevorzugt mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin,

Gemische aus zwei oder mehreren Verbindungen aus einer oder mehreren dieser Klassen sind im Rahmen dieser Ausführungsform ebenso erfindungsgemäß.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Di-hydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen. Ganz besonders bevorzugt ist dabei Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 10 Gew.-%, vorzugsweise 0,01 bis 5,0 Gew.-%, jeweils bezogen auf das anwendungsbereite Mittel, verwendet.

Die nachfolgenden Kombinationen eines erfindungsgemäßen Entwicklers mit ausgewählten Kupplern sind dabei besonders vorteilhaft:
N,N-Dimethyl-3-[(4-aminophenyl)amino]-N-{3-[(4-aminophenyl)amino]-propyl}-1-propanaminium-p-toluolsulfonat, Tetrahydrochlorid und eine oder mehrere Verbindungen, ausgewählt aus 5-Amino-2-methylphenol, 3-Amino-2-chlor-6-methylphenol, 2,4-Dichlor-3-aminophenol, 2-(2,4-Diaminophenoxy)ethanol, 1,3-Bis(2,4-diaminophenoxy)propan, Resorcin, 2-Methylresorcin, 2-Amino-3-hydroxypyridin, 3-Amino-2-methylamino-6-methoxypyridin, 1-Naphthol, 1,5-Dihydroxynaphthalin, 2,7-Dihydroxynaphthalin.

N-Ethyl-N-Methyl-3-[(4-aminophenyl)amino]-N-{3-[(4-aminophenyl)amino]-propyl}-1-propanaminium-p-toluolsulfonat, Tetrahydrochlorid und eine oder mehrere Verbindungen, ausgewählt aus 5-Amino-2-methylphenol, 3-Amino-2-chlor-6-methylphenol, 2,4-Dichlor-3-aminophenol, 2-(2,4-Diaminophenoxy)ethanol, 1,3-Bis(2,4-diaminophenoxy)propan, Resorcin, 2-Methylresorcin, 2-Amino-3-hydroxypyridin, 3-Amino-2-methylamino-6-methoxypyridin, 1-Naphthol, 1,5-Dihydroxynaphthalin, 2,7-Dihydroxynaphthalin.

N-Methyl-N-propyl-3-[(4-aminophenyl)amino]-N-{3-[(4-aminophenyl)amino]-propyl}-1-propanaminium-p-toluolsulfonat, Tetrahydrochlorid und eine oder mehrere Verbindungen, ausgewählt aus 5-Amino-2-methylphenol, 3-Amino-2-chlor-6-methylphenol, 2,4-Dichlor-3-aminophenol, 2-(2,4-Diaminophenoxy)ethanol, 1,3-Bis(2,4-diaminophenoxy)propan, Resorcin, 2-Methylresorcin, 2-Amino-3-hydroxypyridin, 3-Amino-2-methylamino-6-methoxypyridin, 1-Naphthol, 1,5-Dihydroxynaphthalin, 2,7-Dihydroxynaphthalin.

N1,N4-Bis-{3-[(4-aminophenyl)amino]propyl}-N1-methylpiperazinium-p-toluolsulfonat, Hydrochlorid und eine oder mehrere Verbindungen, ausgewählt aus 5-Amino-2-methylphenol, 3-Amino-2-chlor-6-methylphenol, 2,4-Dichlor-3-aminophenol, 2-(2,4-Diaminophenoxy)ethanol, 1,3-Bis(2,4-diaminophenoxy)propan, Resorcin, 2-Methylresorcin, 2-Amino-3-hydroxypyridin, 3-Amino-2-methylamino-6-methoxypyridin, 1-Naphthol, 1,5-Dihydroxynaphthalin, 2,7-Dihydroxynaphthalin.

N,N-Dimethyl-3-[(4-amino-3-methylphenyl)amino]-N-{3-[(4-amino-3-methylphenyl)amino]propyl}-1-propanaminium-p-toluolsulfonat, Tetrahydrochlorid und eine oder mehrere Verbindungen, ausgewählt aus 5-Amino-2-methylphenol, 3-Amino-2-chlor-6-methylphenol, 2,4-Dichlor-3-aminophenol, 2-(2,4-Diaminophenoxy)ethanol, 1,3-Bis(2,4-diaminophenoxy)propan, Resorcin, 2-Methylresorcin, 2-Amino-3-hydroxypyridin, 3-Amino-2-methylamino-6-methoxypyridin, 1-Naphthol, 1,5-Dihydroxynaphthalin, 2,7-Dihydroxynaphthalin.

N-Ethyl-N-methyl-3-[(4-amino-3-methylphenyl)amino]-N-{3-[(4-amino-3-methylphenyl)amino]-propyl}-1-propanaminium-p-toluolsulfonat, Tetrahydrochlorid und eine oder mehrere Verbindungen, ausgewählt aus 5-Amino-2-methylphenol, 3-Amino-2-chlor-6-methylphenol, 2,4-Dichlor-3-aminophenol, 2-(2,4-Diaminophenoxy)ethanol, 1,3-Bis(2,4-diaminophenoxy)propan, Resorcin, 2-Methylresorcin, 2-Amino-3-hydroxypyridin, 3-Amino-2-methylamino-6-methoxypyridin, 1-Naphthol, 1,5-Dihydroxynaphthalin, 2,7-Dihydroxynaphthalin.

N-Methyl-N-propyl-3-[(4-amino-3-methylphenyl)amino]-N-{3-[(4-amino-3-methylphenyl)amino]-propyl}-1-propanaminium-p-toluolsulfonat, Tetrahydrochlorid und eine oder mehrere Verbindungen, ausgewählt aus 5-Amino-2-methylphenol, 3-Amino-2-chlor-6-methylphenol, 2,4-Dichlor-3-aminophenol, 2-(2,4-Diaminophenoxy)ethanol, 1,3-Bis(2,4-diaminophenoxy)propan, Resorcin, 2-Methylresorcin, 2-Amino-3-hydroxypyridin, 3-Amino-2-methylamino-6-methoxypyridin, 1-Naphthol, 1,5-Dihydroxynaphthalin, 2,7-Dihydroxynaphthalin.

Um eine ausgewogene und subtile Nuancenausbildung zu erzielen, ist es erfindungsgemäß vorteilhaft, wenn weitere farbgebende Komponenten im erfindungsgemäßen Mittel enthalten sind. Es kann daher erfindungsgemäß bevorzugt sein, wenn das Mittel mindestens eine weitere farbgebende Komponente enthält, die ausgewählt ist aus zusätzlichen Oxidationsfarbstoffvorprodukten vom Entwicklertyp und/oder direktziehenden Farbstoffen.

Neben den Oxidationsfarbstoffvorprodukten vom Entwickler-Typ gemäß Formel (I) können die erfindungsgemäßen Mittel zusätzlich mindestens eine weitere Entwicklerkomponente enthalten.

Bevorzugte weitere Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, 4-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Besonders bevorzugte zusätzliche Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)-pyrazol sowie deren physiologisch verträglichen Salze.

Die zusätzlichen Entwicklerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 3 Gew.-%.

Direktziehende Farbstoffe können in anionische, kationische und nichtionische direktziehende Farbstoffe unterteilt werden, die entsprechend der Anforderungen der Trägerbasis vom Fachmann ausgewählt und eingesetzt werden.

Bevorzugte anionische direktziehende Farbstoffe sind die unter den internationalen Bezeichnungen bzw. Handelsnamen Bromphenolblau, Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), Basic Blue 99, Basic Brown 16 und Basic Brown 17 sowie Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die erfindungsgemäßen Mittel können neben der Verbindung gemäß Formel (I) auch naturanaloge Farbstoffe enthalten. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Besonders gut als Vorstufen naturanaloger Haarfarbstoffe geeignet sind Derivate des 5,6-Dihydroxyindolins, insbesondere 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure, sowie weiterhin Derivate des 5,6-Dihydroxyindols, insbesondere 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, sowie physiologisch verträgliche Salze der vorstehend genannten Verbindungen.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Peroxodisulfate, Chlorite, Hypochlorite und insbesondere Wasserstoffperoxid oder und/oder eines seiner festen Anlagerungsprodukte an organische oder anorganische Verbindungen in Frage.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht.

In einer weiteren Ausführungsform der vorliegenden Erfindung sind daher Mittel bevorzugt, welche dadurch gekennzeichnet sind, dass sie unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden und wobei ein Container ein Färbemittel (A), welches in einem kosmetischen Träger mindestens ein Oxidationsfarbstoffvorprodukt gemäß Formel (I) enthält, und ein weiterer Container eine Oxidationsmittelzubereitung (B), enthaltend mindestens ein Oxidationsmittel, enthält.

Bevorzugt enthält die Oxidationsmittelzubereitung (B) als Oxidationsmittel Wasserstoffperoxid und/oder eines seiner festen Anlagerungsprodukte an organische oder anorganische Verbindungen, wie Harnstoff, Melamin sowie Natriumborat.

Bevorzugt beträgt die Menge an Oxidationsmittel im anwendungsbereiten Mittel 0,5 bis 12 Gew.-%, bevorzugt 2 bis 10 Gew.-% insbesondere bevorzugt zu 3 bis 6 Gew.-% (berechnet als 100 %-iges H₂O₂), jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Erfindungsgemäß kann aber das Oxidationsfärbemittel auch zusammen mit einem Katalysator auf das Haar aufgebracht werden, der die Oxidation der Farbstoffvorprodukte aktiviert. Solche Katalysatoren sind z. B. bestimmte Enzyme, Iodide, Chinone oder Metallionen.

Weiterhin hat es sich als vorteilhaft erweisen, wenn die Oxidationsmittelzubereitungen mindestens einen Stabilisator oder Komplexbildner enthalten. Gebräuchliche und im Rahmen der vorliegenden Erfindung bevorzugte Komplexbildner und Stabilisatoren sind beispielsweise Polyoxycarbonsäuren, Polyamine, Ethylendiamintetraessigsäure (EDTA), N-Hydroxyethylethylendiamintriessigsäure, Diethylentriaminpentaessigsäure (DTPA), Ethylendiamindibernsteinsäure (EDDS), Hydroxyethyliminodiessigsäure, Nitridodiessigsäure-3-propionsäure, Isoserindiessigsäure, N,N-Di-(2-hydroxyethyl)glycin, N-(1,2-Dicarboxy-2-hydroxyethyl)glycin, N-(1,2-Dicarboxy-2-hydroxyethyl)asparaginsäure oder Nitrilotriessigsäure (NTA), Ethylendiamindiglutarsäure (EDGA), 2-Hydroxypropylendiamindibernsteinsäure (HPDS), Glycinamid-N,N'-dibernsteinsäure (GADS), Ethylendiamin-N-N'-diglutarsäure (EDDG), 2-Hydroxypropylendiamin-N-N'-dibernsteinsäure (HPDDS), Diaminoalkyldi-(sulfobernsteinsäure) (DDS), Ethylendicysteinsäure (EDC), Ethylendiamin-N-N'-bis(ortho-hydroxyphenyl)essigsäure (EDDHA), N-2-Hydroxyethylamin-N,N-diessigsäure, Glyceryliminodiessigsäure, Iminodiessigsäure-N-2-hydroxypropylsulfonsäure, Asparaginsäure-N-carboxymethyl-N-2,5-hydroxypropyl-3-sulfonsäure,β-Alanin-N,N'-diessigsäure, Asparaginsäure-N,N'-diessigsäure, Asparaginsäure-N-monoessigsäure, Dipicolinsäure, sowie deren Salze und/oder Derivate, geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate, Aminophosphonsäuren wie Ethylendiamintetra(methylenphosphonsäure) (EDTMP), Diethylentriaminpenta(methylenphosphonsäure) (DTPMP) sowie deren höhere Homologe, oder Nitrilotri(methylenphosphonsäure), Phosphonopolycarbonsäuren wie 2-Phosphonobutan-1,2,4-tricarbonsäure, Cyclodextrine, sowie Alkalistannate (Natriumstannat), Alkalipyrophosphate (Tetranatriumpyrophosphat, Dinatriumpyrophosphat), Alkaliphosphate (Natriumphosphat), und Phosphorsäure sowie deren Salze.

Die Färbezubereitung und gegebenenfalls Oxidationsmittelzubereitung enthalten weitere Hilfs- und Zusatzstoffe. So hat es sich erfindungsgemäß als bevorzugt erwiesen, wenn die Färbezubereitung und/oder die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Gemäß einer ersten bevorzugten Ausführungsform handelt es sich bei dem Verdickungsmittel um ein anionisches, synthetisches Polymer. Bevorzugte anionische Gruppen sind die Carboxylat- und die Sulfonatgruppe.

Bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichnen Carbopol^{®} im Handel erhältlich. Ebenfalls bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser ersten Ausführungsform kann es weiterhin bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Itaconsäuremono- und -diester, Vinylpyrrolidinon, Vinylether und Vinylester.

Die anionischen Acrylsäure- und/oder Methacrylsäure-Polymerisate oder -Copolymerisate sind in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten.

Bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein bevorzugtes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn^{®} 22 sowie von der Firma National Starch unter den Handelsbezeichnungen Structure^{®} 2001 und Structure^{®} 3001 vertrieben.

Bevorzugte anionische Copolymere sind weiterhin Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxythan, Allylsucrose, Allylpentaerythrit und Methylen-bisacrylamid zum Einsatz kommen. Ein solches Polymer ist in den Handelsprodukten Sepigel^{®}305 und Simulgel^{®} 600 der Firma SEPPIC enthalten. Die Verwendung dieser Verbindungen, die neben der Polymerkomponente eine Kohlenwasserstoffmischung (C₁₃-C₁₄-Isoparaffin beziehungsweise Isohexadecan) und einen nichtionogenen Emulgator (Laureth-7 beziehungsweise Polysorbate-80) enthalten, hat sich im Rahmen der erfindungsgemäßen Lehre als besonders vorteilhaft erwiesen.

Auch Polymere aus Maleinsäureanhydrid und Methylvinylether, insbesondere solche mit Vernetzungen, sind bevorzugte Verdickungsmittel. Ein mit 1,9-Decadien vernetztes Maleinsäure-Methylvinylether-Copolymer ist unter der Bezeichnung Stabileze^{®} QM im Handel erhältlich.

Bevorzugt kann das erfindungsgemäße Mittel zusätzlich mindestens ein anionisches Acrylsäureund/oder Methacrylsäure-Polymerisat oder -Copolymerisat. Bevorzugte Polymerisate dieser Art sind:
- Polymerisate z.B. aus wenigstens 10 Gew.-% Acrylsäure-Niedrigalkylester, 25 bis 70 Gew.-% Methacrylsäure und ggf. bis zu 40 Gew.-% eines weiteren Comonomeren,
- Mischpolymerisate aus 50 bis 75 Gew.-% Ethylacrylat, 25 bis 35 Gew.-% Acrylsäure und 0 bis 25 Gew.-% anderer Comonomeren bekannt. Geeignete Dispersionen dieser Art sind im Handel erhältlich, z.B. unter der Handelsbezeichnung Latekoll^{®} D (BASF).
- Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Gemäß einer weiteren Ausführungsform handelt es sich bei dem Verdickungsmittel um einen kationisches synthetisches Polymer. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen.

### Homopolymere der allgemeinen Formel (HP-1),

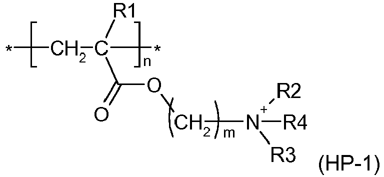

in der R1 = -H oder -CH₃ ist, R2, R3 und R4 unabhängig voneinander ausgewählt sind aus C₁-C₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (HP-1) aufgeführten Monomereinheiten sowie nichtionogenen Monomereinheiten, sind besonders bevorzugte kationische polymere Gelbildner. Im Rahmen dieser Polymeren sind diejenigen erfindungsgemäß bevorzugt, für die mindestens eine der folgenden Bedingungen gilt:
- R1 steht für eine Methylgruppe
- R2, R3 und R4 stehen für Methylgruppen
- m hat den Wert 2,

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloxyethyltrimethylammoniumChlorid) mit der INCI-Bezeichnung Polyquaternium-37.

Copolymere mit Monomereinheiten gemäß Formel (HP-1) enthalten als nichtionogene Monomereinheiten bevorzugt Acrylamid, Methacrylamid, Acrylsäure-C₁-C₄-Alkylester und Methacrylsäure-C₁-C₄-Alkylester. Unter diesen nichtionogenen Monomeren ist das Acrylamid besonders bevorzugt. Auch diese Copolymere können, wie im Falle der Homopolymeren oben beschrieben, vernetzt sein. Ein erfindungsgemäß bevorzugtes Copolymer ist das vernetzte Acrylamid-MethacroyloxyethyltrimethylammoniumChlorid-Copolymer. Solche Copolymere, bei denen die Monomeren in einem Gewichtsverhältnis von etwa 20:80 vorliegen, sind im Handel als ca. 50 %-ige nichtwässrige Polymerdispersion unter der Bezeichnung Salcare^{®} SC 92 erhältlich.

In einer weiteren bevorzugten Ausführungsform werden natürlich vorkommende Verdickungsmittel eingesetzt. Bevorzugte Verdickungsmittel dieser Ausführungsform sind beispielsweise nichtionischen Guargums. Erfindungsgemäß können sowohl modifizierte als auch unmodifizierte Guargums zum Einsatz kommen. Nichtmodifizierte Guargums werden beispielsweise unter der Handelsbezeichnung Jaguar^{®} C von der Firma Rhone Poulenc vertrieben. Erfindungsgemäß bevorzugte modifizierte Guargums enthalten C₁-C₆-Hydroxyalkylgruppen. Bevorzugt sind die Gruppen Hydroxymethyl, Hydroxyethyl, Hydroxypropyl und Hydroxybutyl. Derart modifizierte Guargums sind im Stand der Technik bekannt und können beispielsweise durch Reaktion der Guargums mit Alkylenoxiden hergestellt werden. Der Grad der Hydroxyalkylierung, der der Anzahl der verbrauchten Alkylenoxidmoleküle im Verhältnis zur Zahl der freien Hydroxygruppen der Guargums entspricht, liegt bevorzugt zwischen 0,4 und 1,2. Derart modifizierte Guargums sind unter den Handelsbezeichnungen Jaguar^{®} HP8, Jaguar^{®} HP60, Jaguar^{®} HP120, Jaguar^{®} DC 293 und Jaguar^{®} HP105 der Firma Rhone Poulenc im Handel erhältlich.

Weiterhin geeignete natürliche Verdickungsmittel sind ebenfalls bereits aus dem Stand der Technik bekannt.

Gemäß dieser Ausführungsform bevorzugt sind weiterhin Biosaccharidgums mikrobiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Bevorzugte Hydroxyalkylcellulosen sind insbesondere die Hydroxyethylcellulosen, die unter den Bezeichnungen Cellosize^{®} der Firma Amerchol und Natrosol^{®} der Firma Hercules vertrieben werden. Geeignete Carboxyalkylcellulosen sind insbesondere die Carboxymethylcellulosen, wie sie unter den Bezeichnungen Blanose^{®} von der Firma Aqualon, Aquasorb^{®} und Ambergum^{®} von der Firma Hercules und Cellgon^{®} von der Firma Montello vertrieben werden.

Bevorzugt sind weiterhin Stärke und deren Derivate. Erfindungsgemäß einsetzbar sind natürliche, aus Pflanzen gewonnene Stärken und/oder chemisch oder physikalisch modifizierte Stärken. Eine Modifizierung lässt sich beispielsweise durch Einführung unterschiedlicher funktioneller Gruppen an einer oder mehreren der Hydroxylgruppen der Stärke erreichen. Üblicherweise handelt es sich um Ester, Ether oder Amide der Stärke mit gegebenenfalls substituierten C₁-C₄₀-Resten. Besonders vorteilhaft ist eine mit einer 2-Hydroxypropylgruppe veretherte Maisstärke, wie sie beispielsweise von der Firma National Starch unter der Handelsbezeichnung Amaze^{®} vertrieben wird.

Aber auch nichtionische, vollsynthetische Polymere, wie beispielsweise Polyvinylalkohol oder Polyvinylpyrrolidinon, sind als erfindungsgemäße Verdickungsmittel einsetzbar (beispielsweise Luviskol^{®}; Firma BASF) vertrieben. Derartige nichtionische Polymere ermöglichen, neben ihren hervorragenden verdickenden Eigenschaften, auch eine deutliche Verbesserung des sensorischen Gefühls der resultierenden Zubereitungen.

Als anorganische Verdickungsmittel haben sich Schichtsilikate (polymere, kristalline Natriumdisilicate) als besonders geeignet im Sinne der vorliegenden Erfindung erwiesen. Insbesondere Tone, insbesondere Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate, wie beispielsweise das von der Firma Süd Chemie unter der Handelsbezeichnung Optigel^{®} vertriebene Magnesiumschichtsilikat, sind bevorzugt.

Zur weiteren Steigerung der Leistung der Oxidationsmittelzubereitung können der erfindungsgemäßen Zusammensetzung zusätzlich mindestens eine gegebenenfalls hydratisierte SiO₂-Verbindung zugesetzt. Es kann erfindungsgemäß bevorzugt sein, die gegebenenfalls hydratisierten SiO₂-Verbindung in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder. Hinsichtlich der gegebenenfalls hydratisierten SiO₂-Verbindung unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt. Die gegebenenfalls hydratisierten SiO₂-Verbindungen können in verschiedenen Formen vorliegen. Erfindungsgemäß bevorzugt werden die SiO₂-Verbindung in Form von Kieselgelen (Silicagel) oder besonders bevorzugt als Wasserglas eingesetzt. Diese SiO₂-Verbindung können teilweise in wässriger Lösung vorliegen. Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt. Bevorzugt sind Metasilikate, in denen das Verhältnis zwischen n und der Summe aus m und p bei 1:2 oder darunter liegt. Neben den durch die Summenformel beschriebenen Komponenten können die Wassergläser in geringen Mengen noch weitere Zusatzstoffe, wie beispielsweise Phosphate oder Magnesiumsalze, enthalten.

Vorzugsweise sind die Zubereitung (A) und/oder gegebenenfalls die Oxidationsmittelzubereitung (B) als fließfähigen Zubereitungen konfektioniert.

Vorzugsweise wird den fließfähigen Zubereitungen (A) und/oder (B) weiterhin ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe. Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das C₁₂-C₁₈-Acylsarcosin.

Weiterhin hat es sich als vorteilhaft erwiesen, wenn die erfindungsgemäßen Färbe- und Aufhellmittel weitere, nichtionogene grenzflächenaktive Stoffe, enthalten. Als nichtionische Tenside eignen sich insbesondere C8-C22-Alkylmono- und oligoglycoside und deren ethoxylierte Analoga. Insbesondere die nichtethoxylierten Verbindungen haben sich als besonders geeignet erwiesen. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.

Die anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar. Die Alkylamidoamine werden üblicherweise durch Amidierung natürlicher oder synthetischer Fettsäuren und Fettsäureschnitte mit Dialkylaminoaminen hergestellt und zeichnen sich neben einer guten konditionierenden Wirkung speziell durch ihre gute biologische Abbaubarkeit aus. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar. Bei Esterquats handelt es sich um bekannte Stoffe, die sowohl mindestens eine Esterfunktion als auch mindestens eine quartäre Ammoniumgruppe als Strukturelement enthalten. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für Esterquats.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

Ferner können die erfindungsgemäßen Mittel weitere Wirk-, Hilfs- und Zusatzstoffe enthalten, wie beispielsweise nichtionische Polymere, Silikone, kationische Polymere, zwitterionische und amphotere Polymere, anionische Polymere, weitere Verdickungsmittel, Strukturanten, haarkonditionierende Verbindungen, Lösungsmittel und -vermittler wie Ethanol, Isopropanol, Ethylenglykol, Propylenglykol, Glycerin und Diethylenglykol, faserstrukturverbessernde Wirkstoffe, Farbstoffe zum Anfärben des Mittels, Antischuppenwirkstoffe, Aminosäuren und Oligopeptide , insbesondere Arginin und/oder Serin, Proteinhydrolysate sowie in Form ihrer Fettsäure-Kondensationsprodukte oder gegebenenfalls anionisch oder kationisch modifizierten Derivate, pflanzliche Öle, Lichtschutzmittel, Wirkstoffe wie Panthenol, Pantothensäure, Pantolacton, Allantoin, Pyrrolidinoncarbonsäuren und deren Salze sowie Bisabolol, Ceramide, Vitamine, Provitamine und Vitaminvorstufen, Pflanzenextrakte, Cholesterin, Konsistenzgeber, Fettalkohole, Fette und Wachse, Fettsäurealkanolamide, Quell- und Penetrationsstoffe, Trübungsmittel wie Latex, Styrol/PVP- und Styrol/Acrylamid-Copolymere, Perlglanzmittel wie Ethylenglykolmono- und -distearat sowie PEG-3-distearat, Pigmente, Treibmittel wie Propan-Butan-Gemische, N₂O, Dimethylether, CO₂ und Luft, Antioxidantien.

Die Auswahl dieser weiteren Stoffe wird der Fachmann gemäß der gewünschten Eigenschaften der Mittel treffen.

Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. Die erfindungsgemäßen Mittel können daher zusätzlich noch weitere Blondier- und/oder Bleichmittel enthalten.

Wird neben der Färbung der keratinischen Faser eine starke Aufhellung gewünscht, so ist daher es erfindungsgemäß bevorzugt, wenn zusätzlich eine Blondierzubereitung (C), enthaltend mindestens einen Bleichaktivator, der Mischung aus Oxidationsmittelzubereitung (B) und der Zubereitung (A), enthaltend mindestens ein Oxidationsfarbstoffvorprodukt gemäß Formel (I), beigemischt wird.

Es kann dabei unerheblich sein, ob zunächst eine Mischung aus (A) und (B) hergestellt wird, und anschließend die Blondierzubereitung (C) zugemischt wird, oder ob eine davon verschiedene Reihenfolge der Vermischung der einzelnen Komponenten genutzt wird. Es ist bevorzugt, die einzelnen Zubereitungen in möglichst naher zeitlicher Abfolge zu vermischen und das anwendungsbereite Mittel vorzugsweise zeitnah auf die keratinischen Fasern zu applizieren.

Eine weitere Ausführungsform der vorliegenden Anmeldung ist daher ein Mittel zum Bleichen und Färben keratinischer Fasern, dadurch gekennzeichnet, dass es durch Vermischen von mindestens einer Oxidationsmittelszubereitung (B), enthaltend mindestens ein Oxidationsmittel, ausgewählt aus Wasserstoffperoxid dessen Anlagerungsverbindungen an feste Träger, mindestens einer Blondierzubereitung (C), enthaltend mindestens einen Bleichkraftverstärker, und mindestens einer Zubereitung (A), enthaltend in einem kosmetischen Träger mindestens ein Oxidationsfarbstoffvorprodukt gemäß Formel (I), vor der Anwendung hergestellt wird.

In einer weiteren Ausführungsform ist es bevorzugt, wenn das erfindungsgemäße Färbemittel zusätzlich als Blondierzubereitung (C) mindestens eine anorganische Peroxoverbindung enthält. Vorzugsweise ist die anorganische Peroxoverbindung ausgewählt aus Ammoniumpersulfat, Alkalimetallpersulfaten, Ammoniumperoxomonosulfat, Alkalimetallhydrogenperoxomonosulfaten, Alkalimetallperoxodiphosphaten und Erdalkalimetallperoxiden. Die anorganischen PeroxoVerbindungen sind vorzugsweise in einer Menge von 0,1 bis 25 Gew.-%, insbesondere in einer Menge von 0,5 bis 15 Gew.-%, bezogen auf das Gesamtgewicht des anwendungsbereiten Mittels, enthalten.

Der Einsatz von Persulfatsalzen bzw. Peroxodisulfatsalzen erfolgt in der Regel in Form eines gegebenenfalls entstaubten Pulvers oder eines in Form gepressten Formkörpers.

Obwohl prinzipiell keine Einschränkungen hinsichtlich der Formulierung der Blondierzubereitung (C) bestehen, hat es sich erfindungsgemäß als bevorzugt erwiesen, wenn die Zubereitung (C) wasserfrei formuliert ist.

Wasserfrei im Sinne der vorliegenden Erfindung bedeutet einen Wassergehalt bezogen auf die Zubereitung (C) von weniger als 5 Gew.-%, insbesondere von weniger als 2 Gew.-%. Blondierzubereitungen, die weniger als 0,1 Gew.-% Wasser, enthalten können erfindungsgemäß ganz besonders bevorzugt sein. Die Zubereitung (C) ist vorzugsweise als Pulver oder als wasserfreie Paste formuliert.

In einer weiteren, bevorzugten Ausführungsform kann das Mittel in der Zubereitung (C) als Bleichkraftverstärker mindestens ein kationisches Pyridinium-Derivat enthalten. Insbesondere sind erfindungsgemäße Mittel bevorzugt, die als kationisches Pyridinium-Derivat mindestens eine Verbindung aus 2-Acetyl-1-methylpyridinium-p-toluolsulfonat und/oder 4-Acetyl-1-methylpyridinium-p-toluolsulfonat und/oder N-Methyl-3,4-dihydroisochinolinium-p-toluolsulfonat.

Eine erfindungsgemäß bevorzugte Ausführungsform der vorliegenden Erfindung besteht darin, dass das anwendungsbereite Mittel einen pH- zwischen 7 und 11, insbesondere zwischen 8 und 10,5, insbesondere bevorzugt zwischen 8,5 und 10,0, besitzt.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Bei den pH-Werten im Sinne der vorliegenden Erfindung handelt es sich um pH-Werte, die bei einer Temperatur von 22 °C gemessen wurden.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden ausgewählt aus der Gruppe 2-Aminoethan-1-ol (Monoethanolamin), 2-Amino-2-methylpropan-1-ol, 2-Amino-2-methyl-propan-1,3-diol und Triethanolamin. Anorganische Alkalisierungsmittel werden bevorzugt ausgewählt aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Ganz besonders bevorzugt sind Natriumhydroxid und/oder Kaliumhydroxid. Die basischen Aminosäuren werden bevorzugt ausgewählt aus L-Arginin, D-Arginin, D/L-Arginin, L-Lysin, D-Lysin, D/L-Lysin, besonders bevorzugt L-Arginin, D-Arginin, D/L-Arginin. Schließlich ist ein weiteres bevorzugtes Alkalisierungsmittel Ammoniak.

Bevorzugt werden die Alkalisierungsmittel in einer Menge von 0,05 bis 10 Gew.-%, insbesondere von 0,5 bis 5 Gew.-%, jeweils bezogen auf das Gesamtgewicht des anwendungsbereiten Mittels, enthalten.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Eine bevorzugte Darreichungsform des erfindungsgemäßen Mittels ist eine Verpackungseinheit (Kit-of-Parts), welche in getrennt voneinander konfektionierten Containern
- in einem Container A mindestens eine Zubereitung (A) enthält, enthaltend in einem kosmetischen Träger mindestens ein Oxidationsfarbstoffvorprodukt gemäß der Formel (I), und
- in einem Container B mindestens eine Oxidationsmittelzubereitung (B), enthaltend in einem kosmetischen Träger mindestens ein Oxidationsmittel, enthält.
   Wird ein besonders starker Aufhelleffekt gewünscht, so ist eine bevorzugte weitere Darreichungsform des erfindungsgemäßen Mittels eine Verpackungseinheit (Kit-of-Parts), welche in getrennt voneinander konfektionierten Containern
   -in einem Container A mindestens eine Zubereitung (A), enthaltend in einem kosmetischen Träger mindestens ein Oxidationsfarbstoffvorprodukt gemäß der Formel (I),
- in einem Container B mindestens eine Oxidationsmittelzubereitung (B), enthaltend mindestens ein Oxidationsmittel, und
- in einem Container C mindestens eine Blondierzubereitung (C), enthaltend mindestens einen Bleichkraftverstärker, enthält.

Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Applikationshilfe, wie beispielsweise ein Kamm oder ein Pinsel, und/oder eine persönliche Schutzausrüstung, wie beispielsweise Einweg-Handschuhe dem Kit beigefügt ist.

Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Das eigentliche Haarfärbemittel wird zweckmäßigerweise unmittelbar vor der Anwendung durch Mischung der Zubereitungen (A) mit (B) sowie gegebenenfalls (C) hergestellt. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein weiterer Gegenstand der vorliegenden Erfindung ist daher ein Verfahren zur Färben und gegebenenfalls Aufhellen menschlicher Haare, bei dem ein erfindungsgemäßes Mittel gemäß obiger Vorgaben auf das Haar aufgetragen wird, für eine Einwirkzeit von 5 bis 45 Minuten, bevorzugt von 8 bis 35 Minuten, auf dem Haar belassen wird, und aus dem Haar ausgespült oder mit einem Shampoo ausgewaschen wird.

Während der Einwirkzeit des Mittels auf der Faser kann es vorteilhaft sein, den Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Haarfärbung am lebenden Probanden, durch die Körpertemperatur des Probanden erfolgen. Bei letzterer Möglichkeit wird üblicherweise die zu färbende Partie mit einer Haube abgedeckt. Insbesondere liegt die Temperatur während der Einwirkzeit zwischen 10 °C und 45 °C, insbesondere zwischen 20 °C und 40 °C. Die erfindungsgemäßen Färbemittel ergeben bereits bei physiologisch verträglichen Temperaturen von unter 45°C intensive Färbungen. Sie eignen sich deshalb besonders zum Färben von menschlichen Haaren.

Ein weiterer Gegenstand der vorliegenden Erfindung ist die Verwendung eines erfindungsgemäßen Mittels in oxidativen Färbemitteln für menschliche Haare zur Verbesserung der Grauabdeckung, der Egalisierung, der Farbintensität, der Haltbarkeit und/oder der Farbigkeit der Färbeergebnisse.

Bezüglich weiterer bevorzugter Ausführungsformen der erfindungsgemäßen Verfahren und Verwendungen gilt mutatis mutandis das zu den erfindungsgemäßen Mitteln Gesagte.

Schließlich betrifft ein weiterer Gegenstand der vorliegenden Erfindung Verbindungen gemäß Formel (I) des ersten Erfindungsgegenstands. Bezüglich weiterer bevorzugter Ausführungsformen dieser Verbindungen gilt mutatis mutandis das zu den erfindungsgemäßen Mitteln Gesagte.

### Beispiele- Synthesebeispiele

### Synthesebeispiel 1: N,N-Dimethyl-3-[(4-aminophenyl)amino]-N-{3-[(4-aminophenyl)amino]-propyl}-1-propanaminium-p-toluolsulfonat, Tetrahydrochlorid (E 1)

### 1.1. Synthese von N1-Methyl-N3-(4-nitrophenyl)-N1-{3-[(4-nitrophenyl)amino]propyl}-1,3-propandiamin

50,0 g (0,32 mol) 4-Chlornitrobenzol wurden in 100 ml Dimethylsulfoxid vorgelegt und auf 80°C erwärmt (klare Lösung). Dann wurden 15,6 g (0,16 mol) Natriumcarbonat hinzugefügt. Bei dieser Temperatur wurden anschließend 23,2 g (0,16 mol) N1-(3-Aminopropyl)-N1-methyl-1,3-propandiamin zugetropft. Nach Beendigung des Zutropfens wurde das Reaktionsgemisch für 12 h bei 120°C gerührt. Nach dem Abkühlen wurde auf 1 I Wasser gegossen, die wässrige Phase wurde 3 mal mit je 200 ml Essigsäureethylester extrahiert. Danach wurde die organische Phase mit Natriumsulfat getrocknet und am Rotationsverdampfer im Vakuum eingeengt. Das Produkt verblieb als Rückstand in Form eines hochviskosen Öls. Ausbeute: 48,1 g (78,2 %); ¹H-NMR (400 MHz, DMSO-d₆): δ [ppm] = 1,71 (m, 4H); 2,17 (s, 3H); 2,40 (t, 4H); 3,18 (m, 4H); 6,61 (d, 4H); 7,27 (br., 2 x NH); 7,98 (d, 4H).

### 1.2. Synthese von N,N-Dimethyl-3-[(4-nitrophenyl)amino]-N-{3-[(4-nitrophenyl)amino]propyl}-1-propanaminium-p-toluolsulfonat

40,0 g (0,10 mol) N1-Methyl-N3-(4-nitrophenyl)-N1-{3-[(4-nitrophenyl)amino]propyl}-1,3-propandiamin aus Stufe 1.1. wurden zusammen mit 20,5 g (0,11 mol) p-Toluolsulfonsäuremethylester in 500 ml Toluol für 6 h unter Rückfluss erhitzt. Das zu quaternierende Edukt löste sich zunächst in der Kälte nicht in Toluol, ging aber beim Erhitzen langsam in Lösung. Im Laufe der Reaktion schied sich das Produkt in Form einer öligen Substanz ab, welche durch Abdekantieren von der überstehenden Lösung abgetrennt wurde. Das Öl wurde nochmals mit 300 ml Toluol ausgerührt und anschließend im Vakuum getrocknet. Ausbeute: 14,6 g (24,7 %); ¹H-NMR (400 MHz, DMSO-d₆): δ [ppm] = 2,03 (m, 4H); 2,30 (s, 3H); 3,08 (s, 6H); 3,22 (m, 4H); 3,43 (m, 4H); 6,67 (d, 4H); 7,16 (d, 2H); 7,59 (d, 2H); 8,01 (d, 4H).

### 1.3. Synthese von N,N-Dimethyl-3-[(4-aminophenyl)amino]-N-{3-[(4-aminophenyl)amino]-propyl}-1-propanaminium-p-toluolsulfonat, Tetrahydrochlorid (E 1):

54,0 g (0,09 mol) N,N-Dimethyl-3-[(4-nitrophenyl)amino]-N-{3-[(4-nitrophenyl)amino]propyl}-1-propanaminium-p-toluolsulfonat aus Stufe 1.2. wurden in 900 ml Etanol und 100 ml dest. Wasser gelöst. Als Katalysator wurden 0,5 g Palladium auf Kohle (5 %) hinzugegeben, dann wurde das Gemisch im Autoklaven bei Raumtemperatur und einem Wasserstoffdruck von 10 bar hydriert. Nach Beendigung der Reduktion wurde vom Katalysator abfiltriert und die Aminoverbindung durch Zugabe von 150 ml halbkonzentrierter Salzsäure zum Filtrat in das Hydrochloridsalz überführt. Danach wurde das Filtrat komplett eingeengt, wobei das Produkt in Form eines rötlichen, kristallinen Feststoffes zurückblieb. Ausbeute: 26,9 g (50,9 %); ¹H-NMR (400 MHz, DMSO-d₆): δ [ppm] = 2,14 (m, 4H); 2,30 (s, 3H); 3,09 (s, 6H); 3,24 (m, 4H); 3,52 (m, 4H); 7,12 (d, 2H); 7,19 (d, 4H); 7,34 (d, 4H); 7,51 (d, 2H).

### Synthesebeispiel 2: N-Ethyl-N-Methyl-3-[(4-aminophenyl)amino]-N-{3-[(4-aminophenyl)-amino]propyl}-1-propanaminium-p-toluolsulfonat, Tetrahydrochlorid (E 2)

### 2.1. Synthese von N1-Methyl-N3-(4-nitrophenyl)-N1-{3-[(4-nitrophenyl)amino]propyl}-1,3-propandiamin

vgl. Synthesebeispiel 1, Stufe 1.1.

### 2.2. Synthese von N-Ethyl-N-Methyl-3-[(4-nitrophenyl)amino]-N-{3-[(4-nitrophenyl)amino]-propyl}-1-propanaminium-p-toluolsulfonat

40,0 g (0,10 mol) N1-Methyl-N3-(4-nitrophenyl)-N1-{3-[(4-nitrophenyl)amino]propyl}-1,3-propandiamin aus Stufe 2.1. wurden zusammen mit 20,5 g (0,11 mol) p-Toluolsulfonsäure-ethylester in 500 ml Toluol für 6 h unter Rückfluss erhitzt. Im Laufe der Reaktion separierte sich das Produkt in Form einger öligen Substanz. Dieses Öl wurde durch Abdekantieren der überstehenden Flüssigkeit abgetrennt und mit 500 ml Toluol ausgerührt. Danach wurde das Öl mechanisch aus dem Kolben entfernt, getrocknet und gemörsert. Ausbeute: 43,0 g (70,8 %); ¹H-NMR (400 MHz, DMSO-d₆): δ [ppm] = 1,19 (t, 3H); 1,96 (m, 4H); 2,29 (s, 3H); 2,98 (s, 3H); 3,27 (m, 4H); 3,33 (m, 6H); 6,68 (d, 4H); 7,17 (d, 2H); 7,39 (br., 2 x NH); 7,56 (d, 2H); 8,01 (d, 4H).

### 2.3. Synthese von N-Ethyl-N-Methyl-3-[(4-aminophenyl)amino]-N-{3-[(4-aminophenyl)amino]-propyl}-1-propanaminium-p-toluolsulfonat, Tetrahydrochlorid (E 2)

37,8 g (64 mmol) N-Ethyl-N-Methyl-3-[(4-nitrophenyl)amino]-N-{3-[(4-nitrophenyl)amino]-propyl}-1-propanaminium-p-toluolsulfonat aus Stufe 2.2. wurden in 900 ml Etanol und 100 ml dest. Wasser gelöst. Als Katalysator wurden 0,5 g Palladium auf Kohle (5 %) hinzugegeben, dann wurde das Gemisch im Autoklaven bei Raumtemperatur und einem Wasserstoffdruck von 15 bar hydriert. Nach Beendigung der Reduktion wurde vom Katalysator abfiltriert und die Aminoverbindung durch Zugabe von 150 ml halbkonzentrierter Salzsäure zum Filtrat in das Hydrochloridsalz überführt. Danach wurde das Filtrat komplett eingeengt, wobei das Produkt in Form eines braunen, kristallinen Feststoffes zurückblieb. Ausbeute: 27,8 g (75,1 %); ¹H-NMR (400 MHz, DMSO-d₆): δ [ppm] = 1,31 (t, 3H); 2,28 (m, 4H); 2,36 (s, 3H); 3,09 (s, 3H); 3,44 (m, 6H); 3,54 (m, 4H); 7,29 (d, 2H); 7,59 (m, 8H); 7,62 (d, 2H).

### Synthesebeispiel 3: N-Methyl-N-propyl-3-[(4-aminophenyl)amino]-N-{3-[(4-aminophenyl)-amino]propyl}-1-propanaminium-p-toluolsulfonat, Tetrahydrochlorid (E3)

### 3.1. Synthese von N1-Methyl-N3-(4-nitrophenyl)-N1-{3-[(4-nitrophenyl)amino]propyl}-1,3-propandiamin

vgl. Synthesebeispiel 1, Stufe 1.1.

### 3.2. Synthese von N-Allyl-N-methyl-3-[(4-nitrophenyl)amino]-N-{3-[(4-nitrophenyl)amino]-propyl}-1-propanaminium-p-toluolsulfonat

40,0 g (0,10 mol) N1-Methyl-N3-(4-nitrophenyl)-N1-{3-[(4-nitrophenyl)amino]propyl}-1,3-propandiamin aus Stufe 3.1. wurden zusammen mit 29,4 g (0,11 mol) p-Toluolsulfonsäureallylester in 500 ml Toluol für 6 h unter Rückfluss erhitzt. Im Laufe der Reaktion separierte sich das Produkt in Form einger öligen Substanz. Dieses Öl wurde durch Abdekantieren der überstehenden Flüssigkeit abgetrennt und mit 300 ml Toluol ausgerührt. Dann wurde das Produkt getrocknet und gemörsert. Ausbeute: 43,9 g (70,0 %); ¹H-NMR (400 MHz, DMSO-d₆): δ [ppm] = 1,98 (m, 4H); 2,29 (s, 3H); 3,00 (s, 3H); 3,22 (m, 4H); 3,38 (m, 4H); 3,99 (d, 2H); 5,15 (dd, 1H); 5,55 (dd, 1H); 6,04 (m, 1H); 6,67 (d, 4H); 7,12 (d, 2H); 7,53 (d, 2H); 7,98 (d, 4H).

### 3.3. Synthese von N-Methyl-N-propyl-3-[(4-aminophenyl)amino]-N-{3-[(4-aminophenyl)amino]-propyl}-1-propanaminium-p-toluolsulfonat, Tetrahydrochlorid (E3)

37,8 g (64,3 mmol) N-Allyl-N-methyl-3-[(4-nitrophenyl)amino]-N-{3-[(4-nitrophenyl)amino]-propyl}-1-propanaminium-p-toluolsulfonat aus Stufe 3.2.wurden in 900 ml Etanol und 100 ml dest. Wasser gelöst. Als Katalysator wurden 0,5 g Palladium auf Kohle (5 %) hinzugegeben, dann wurde das Gemisch im Autoklaven bei Raumtemperatur und einem Wasserstoffdruck von 15 bar hydriert. Nach Beendigung der Reduktion wurde vom Katalysator abfiltriert und die Aminoverbindung durch Zugabe von 150 ml halbkonzentrierter Salzsäure zum Filtrat in das Tetrahydrochlorid überführt. Danach wurde das Filtrat komplett eingeengt, wobei das Produkt in Form eines hellbraunen, kristallinen Feststoffes zurückblieb. Ausbeute: 15,4 g (39,7 %).

### Synthesebeispiel 4: N1,N4-Bis-{3-[(4-aminophenyl)amino]propyl}-N1-methylpiperazinium-p-toluolsulfonat, Hydrochloridsalz (E 4)

### 4.1. Synthese von N1,N4-Bis-(4-nitrophenyl)-1,4-piperazindipropanamin

28,2 g (0,20 mol) 4-Fluornitrobenzen, 21,0 g (0,10 mol) 1,4-Bis(3-aminopropyl)piperazin und 61,8 g (0,61 mol) Triethylamin wurden zusammen in 200 ml Dimethylsulfoxid für 10 h bei 80°C gerührt. Dann wurden 40 ml Wasser zugesetzt und nochmals für weitere 3 h bei 80°C gerührt (Niederschlagsbildung). Nach dem Abkühlen wurde auf 1 I Wasser gegossen. Der ausgefallene Feststoff wurde filtriert, mit Wasser nachgewaschen und im Vakuum getrocknet. Ausbeute: 40,2 g (90,7 %); ¹H-NMR (400 MHz, DMSO-d₆): δ [ppm] = 1,69 (m, 4H); 2,38 (m, 12 H); 3,18 (m, 4H); 6,63 (d, 4H); 7,32 (br., 2 x NH); 7,99 (d, 4H).

### 4.2. Synthese von N1,N4-Bis-{3-[(4-nitrophenyl)amino]propyl}-N1-methylpiperazinium-p-toluolsulfonat

35,0 g (0,079 mol) N1,N4-Bis-(4-nitrophenyl)-1,4-piperazindipropanamin aus Stufe 6.1. wurden zusammen mit 16,2 g (0,087 mol) p-Toluolsulfonsäuremethylester in 400 ml Toluol für 6 h unter Rückfluss erhitzt. Im Laufe der Reaktion schied sich das Produkt in Form einer öligen Substanz ab, welche durch Abdekantieren von der überstehenden Lösung abgetrennt wurde. Das Öl erstarrte beim weiteren Abkühlen in glasartiger Form, war nach dem Trocknen pulverisierbar, so dass das Produkt in Form eines gelben Feststoffes vorlag. Ausbeute: 45,1 g (90,7 %).

### 4.3. Synthese von N1,N4-Bis-{3-[(4-aminophenyl)amino]propyl}-N1-methylpiperazinium-p-toluolsulfonat, Hydrochloridsalz (E 4)

38,4 g (61,1 mmol) N1,N4-Bis-{3-[(4-nitrophenyl)amino]propyl}-N1-methylpiperazinium-p-toluolsulfonat aus Stufe 4.2. wurden wurden in 900 ml Etanol und 100 ml dest. Wasser gelöst. Als Katalysator wurden 0,5 g Palladium auf Kohle (5 %) hinzugegeben, dann wurde das Gemisch im Autoklaven bei Raumtemperaturn und einem Wasserstoffdruck von 10 bar hydriert. Nach Beendigung der Reduktion wurde vom Katalysator abfiltriert und die Aminoverbindung durch Zugabe von 150 ml halbkonzentrierter Salzsäure zum Filtrat in das Hydrochloridsalz überführt. Danach wurde das Filtrat komplett eingeengt, wobei das braune, wachsartige Produkt zurückblieb. Ausbeute: 20,8 g (53,2 %).

### Synthesebeispiel 5: N,N-Dimethyl-3-[(4-amino-3-methylphenyl)aminol-N-{3-[(4-amino-3-methylphenyl)amino]propyl}-1-propanaminium-p-toluolsulfonat, Tetrahydrochlorid (E 5)

### 5.1. Synthese von N1-Methyl-N3-(3-methyl-4-nitrophenyl)-N1-{3-[(3-methyl-4-nitrophenyl)-amino]propyl}-1,3-propandiamin

50,0 g (0,32 mol) 5-Fluor-2-nitrotoluol wurden bei 60 °C mit 17,1 g (0,16 mol) Natriumcarbonat versetzt. Dann wurden vorsichtig 23,7 g (0,16 mol) 3,3'-Diamino-N-methyldipropylamin zugetropft (exotherme Reaktion). Das resultierende Gemisch wurde für 12 h auf 100°C erhitzt. Nach dem Abkühlen wurde das Reaktionsgemisch mit 100 mol Toluol versetzt, die hierbei ausfallenden anorganischen Salze wurden abfiltriert. Anschließend wurde die organische Phase komplett am Rotationsverdamper im Vakuum eingeengt, wobei als Rückstand das Produkt in Form eines gelborangen Öls erhalten wurde. Ausbeute: 52,5 g (78,4 %); ¹H-NMR (400 MHz, DMSO-d₆): δ [ppm] = 1,71 (m, 4H); 2,18 (s, 3H); 2,40 (m, 4H); 2,55 (s, 6H); 3,17 (m, 4H); 6,45 (s, 2H); 6,48 (d, 2H); 7,08 (br., 2 x NH); 7,92 (d, 2H).

### 5.2. Synthese von N,N-Dimethyl-3-[(3-methyl-4-nitrophenyl)amino]-N-{3-[(3-methyl-4-nitrophenyl)amino]propyl}-1-propanaminium-p-toluolsulfonat

57,0 g (0,14 mol) N1-Methyl-N3-(3-methyl-4-nitrophenyl)-N1-{3-[(3-methyl-4-nitrophenyl)-amino]propyl}-1,3-propandiamin aus Stufe 5.1. wurden zusammen mit 28,4 g (0,15 mol) p-Toluolsulfonsäuremethylester in 500 ml Toluol für 6 h unter Rückfluss erhitzt. Im Laufe der Reaktion separierte sich das Produkt in Form einer öligen Substanz. Dieses Öl wurde durch Abdekantieren der überstehenden Flüssigkeit abgetrennt, mit 300 ml Toluol ausgerührt und getrocknet. Beim weiteren Abkühlen erstarrte das Öl zunächst glasartig und ließ sich nach dem Trocknen pulverisieren, so dass das Produkt in Form eines braunen Pulvers vorlag. Ausbeute: 58,4 g (70,8 %);¹H-NMR (400 MHz, DMSO-d₆): δ [ppm] = 1,98 (m, 4H); 2,29 (s, 3H); 2,51 (s, 6H); 3,17 (s, 6H); 3,21 (m, 4H); 3,40 (m, 4H); 6,51 (s, 2H); 6,52 (d, 2H); 7,17 (d, 2H); 7,59 (d, 2H); 7,98 (d, 2H).

### 5.3. Synthese von N,N-Dimethyl-3-[(4-amino-3-methylphenyl)amino]-N-{3-[(4-amino-3-methylphenyl)amino]propyl}-1-propanaminium-p-toluolsulfonat, Tetrahydrochlorid (E 5)

58,0 g (86,7 mmol) N,N-Dimethyl-3-[(3-methyl-4-nitrophenyl)amino]-N-{3-[(3-methyl-4-nitrophenyl)amino]propyl}-1-propanaminium-p-toluolsulfonat aus Stufe 5.2.wurden in 900 ml Etanol und 100 ml dest. Wasser gelöst. Als Katalysator wurden 0,5 g Palladium auf Kohle (5 %) hinzugegeben, dann wurde das Gemisch im Autoklaven bei Raumtemperatur und einem Wasserstoffdruck von 15 bar hydriert. Nach Beendigung der Reduktion wurde vom Katalysator abfiltriert und die Aminoverbindung durch Zugabe von 150 ml halbkonzentrierter Salzsäure zum Filtrat in das Hydrochloridsalz überführt. Danach wurde das Filtrat komplett eingeengt, wobei das Produkt in Form eines hellroten, kristallinen Feststoffes zurückblieb. Ausbeute: 52,0 g (87,8 %); ¹H-NMR (400 MHz, DMSO-d₆): δ [ppm] = 2,18 (m, 4H); 2,33 (s, 6H); 2,41 (s, 3H); 3,14 (s, 6H); 3,30 (m, 4H); 3,59 (m, 4H); 7,19 (d, 2H); 7,30-7,55 (m, 8H).

### Synthesebeispiel 6: N-Ethyl-N-methyl-3-[(4-amino-3-methylphenyl)aminol-N-{3-[(4-amino-3-methylphenyl)amino]propyl}-1-propanaminium-p-toluolsulfonat, Tetrahydrochlorid (E 6)

### 6.1. Synthese von N1-Methyl-N3-(3-methyl-4-nitrophenyl)-N1-{3-[(3-methyl-4-nitrophenyl)-amino]propyl}-1,3-propandiamin

vgl. Synthesebeispiel 5, Stufe 5.1.

### 6.2. Synthese von N-Ethyl-N-methyl-3-[(3-methyl-4-nitrophenyl)amino]-N-{3-[(3-methyl-4-nitrophenyl)amino]propyl}-1-propanaminium-p-toluolsulfonat

52,0 g (0,13 mol) N1-Methyl-N3-(3-methyl-4-nitrophenyl)-N1-{3-[(3-methyl-4-nitrophenyl)-amino]-propyl}-1,3-propandiamin aus Stufe 6.1. wurden zusammen mit 28,0 g (0,14 mol) p-Toluolsulfonsäureethylester in 500 ml Toluol für 6 h unter Rückfluß erhitzt. Im Laufe der Reaktion separierte sich das Produkt in Form einer öligen Substanz. Dieses Öl wurde durch Abdekantieren der überstehenden Flüssigkeit abgetrennt und getrocknet. Beim weiteren Abkühlen erstarrte das Öl zunächst glasartig und ließ sich nach dem Trocknen pulverisieren, so dass das Produkt in Form eines braunen Pulvers vorlag. Ausbeute: 60,9 g (79,1 %); ¹H-NMR (400 MHz, DMSO-d₆): δ [ppm] = 1,21 (t, 3H); 1,97 (m, 4H); 2,29 (s, 3H); 2,99 (s, 3H); 3,23 (m, 4H); 3,33 (m, 4H); 3,43 (q, 2H); 6,50 (dd, 2H); 6,55 (dd, 2H); 7,18 (d, 2H); 7,20 (br. 2 x NH); 7,54 (d, 2H); 7,96 (d, 2H).

### 6.3. Synthese von N-Ethyl-N-methyl-3-[(4-amino-3-methylphenyl)amino]-N-{3-[(4-amino-3-methylphenyl)amino]propyl}-1-propanaminium-p-toluolsulfonat, Tetrahydrochlorid (E 6)

50,5 g (82 mmol) N-Ethyl-N-methyl-3-[(3-methyl-4-nitrophenyl)amino]-N-{3-[(3-methyl-4-nitrophenyl)amino]propyl}-1-propanaminium-p-toluolsulfonat aus Stufe 6.2.wurden in 900 ml Etanol und 100 ml dest. Wasser gelöst. Als Katalysator wurden 0,5 g Palladium auf Kohle (5 %) hinzugegeben, dann wurde das Gemisch im Autoklaven bei Raumtemperatur und einem Wasserstoffdruck von 10 bar hydriert. Nach Beendigung der Reduktion wurde vom Katalysator abfiltriert und die Aminoverbindung durch Zugabe von 150 ml halbkonzentrierter Salzsäure zum Filtrat in das Tetrahydrochlorid überführt. Danach wurde das Filtrat komplett eingeengt, wobei das Produkt in Form eines beigen, kristallinen Feststoffes zurückblieb. Ausbeute: 21,6 g (41,9 %); ¹H-NMR (400 MHz, DMSO-d₆): δ [ppm] = 1,32 (t, 3H); 2,27 (m, 4H); 2,36 (s, 3H); 2,48 (s, 6H); 3,09 (s, 3H); 3,45 (m, 6H); 3,55 (m, 4H); 7,27 (d, 2H); 7,47 (dd, 2H); 7,58 (dd, 2H); 7,59 (d, 2H); 7,63 (d, 2H).

### Synthesebeispiel 7: N-Methyl-N-Propyl-3-[(4-amino-3-methylphenyl)aminol-N-{3-[(4-amino-3-methylphenyl)amino]propyl}-1-propanaminium-p-toluolsulfonat, Tetrahydrochlorid (E 7)

### 7.1. Synthese von N1-Methyl-N3-(3-methyl-4-nitrophenyl)-N1-{3-[(3-methyl-4-nitrophenyl)-amino]propyl}-1,3-propandiamin

vgl. Synthesebeispiel 5, Stufe 5.1.

### 7.2. Synthese von N-Allyl-N-methyl-3-[(3-methyl-4-nitrophenyl)amino]-N-{3-[(3-methyl-4-nitrophenyl)amino]propyl}-1-propanaminium-p-toluolsulfonat

60,2 g (0,14 mol) N1-Methyl-N3-(3-methyl-4-nitrophenyl)-N1-{3-[(3-methyl-4-nitrophenyl)-amino]-propyl}-1,3-propandiamin aus Stufe 7.1. wurden zusammen mit 34,0 g (0,16 mol) p-Toluolsulfonsäureallylester in 500 ml Toluol für 6 h unter Rückfluss erhitzt. Im Laufe der Reaktion separierte sich das Produkt in Form einer öligen Substanz. Dieses Öl wurde durch Abdekantieren der überstehenden Flüssigkeit abgetrennt und getrocknet. Beim weiteren Abkühlen erstarrte das Öl zunächst glasartig und ließ sich nach dem Trocknen pulverisieren, so dass das Produkt in Form eines braunen Pulvers vorlag. Ausbeute: 66,0 g (72,5 %).

### 7.3. Synthese von N-Methyl-N-propyl-3-[(4-amino-3-methylphenyl)amino]-N-{3-[(4-amino-3-methylphenyl)amino]propyl}-1-propanaminium-p-toluolsulfonat, Tetrahydrochlorid (E 7)

56,0 g (89,2 mmol) N-Allyl-N-methyl-3-[(3-methyl-4-nitrophenyl)amino]-N-{3-[(3-methyl-4-nitrophenyl)amino]propyl}-1-propanaminium-p-toluolsulfonat aus Stufe 7.2. wurden in 900 ml Etanol und 100 ml dest. Wasser gelöst. Als Katalysator wurden 0,5 g Palladium auf Kohle (5 %) hinzugegeben, dann wurde das Gemisch im Autoklaven bei Raumtemperatur und einem Wasserstoffdruck von 15 bar hydriert. Nach Beendigung der Reduktion wurde vom Katalysator abfiltriert und die Aminoverbindung durch Zugabe von150 ml halbkonzentrierter Salzsäure zum Filtrat in das Hydrochloridsalz überführt. Danach wurde das Filtrat komplett eingeengt, wobei das Produkt in Form eines braunen, kristallinen Feststoffes zurückblieb. Ausbeute: 18,1 g (37,6 %).

### Beispiele - Färbebeispiele

### Herstellung der Färbecreme

Es wurden die folgende Färbecreme hergestellt:

| | |
|---|---|
| Hydrenol D | 8,5 Gew.-% |
| Lorol tech. | 2,0 Gew.-% |
| Texapon NSO UP | 20,0 Gew.-% |
| Dehyton K | 12,5 Gew.-% |
| Eumulgin B2 | 0,75 Gew.-% |
| Natriumsulfit | 1,0 Gew.-% |
| Ammoniumsulfat | 1,0 Gew.-% |
| Entwicklerkomponente E1 | 3 mmol |
| Kupplerkomponente | 3 mmol |
| Wasser | ad 100 Gew.-% |

**Eingesetzte Rohstoffe:**

| | |
|---|---|
| Hydrenol D | C₁₂-C₁₈-Fettalkohol, (INCI-Bezeichnung: Cetearylalcohol; Cognis) |
| Lorol, techn. | C₁₂-/C₁₈-Fettalkohol, (INCI-Bezeichnung: Cetearylalcohol; Cognis) |
| Texapon NSO UP | Laurylalkohol-diglykolethersulfat, Na-Salz (28% Lösung) (INCI-Bezeichnung: Sodium Laureth Sulfate; Cognis) |
| Dehyton K | Cocoyl-betain (INCI-Bezeichnung: Cocamidopropyl betaine; Cognis) |
| Eumulgin B2 | C₁₆-/C₁₈-Fettalkohol, ethoxyliert (20 EO) (INCI-Bezeichnung: Ceteareth-10; Cognis) |

Hydrenol D und Lorol, techn. wurden zusammen mit Texapon NSO-UP, Dehyton K und Eumulgin B2 bei 80°C aufgeschmolzen. Dann wurde die Schmelze mit dem in einem Teil des Wassers gelösten Natriumsulfit und Ammoniumsulfat emulgiert. Der erfindungsgemäße Entwickler wurde in Propylenglykol und einem weiteren Teil der angegebenen Wassermenge unter Erhitzen gelöst und unter Rühren hinzugegeben. Der Kuppler wurde ebenfalls in einem Teil der angegebenen Wassermenge gelöst und unter Rühren hinzugegeben. Dann wurde die Formulierung auf 100 % mit Wasser aufgefüllt und kalt gerührt.

Die auf diese Weise erhaltene Färbecreme wurde im Verhältnis 2:1 mit der folgenden Entwicklerdispersion mit einem Wasserstoffperoxidgehalt von 3 % vermischt.

| | |
|---|---|
| Dipicolinsäure | 0,1 Gew.-% |
| Natriumpyrophosphat | 0,03 Gew.-% |
| Turpinal SL | 1,50 Gew.-% |
| Texapon N28 | 2,00 Gew.-% |
| Acrysol 22 | 0,60 Gew.-% |
| Wasserstoffperoxid, 50 Gew.-%ig | 6,00 Gew.-% |
| Natronlauge, 45 Gew.-%ig | 0,80 Gew.-% |
| Wasser | ad 100 Gew.-% |

**Eingesetzte Rohstoffe:**

| | |
|---|---|
| Turpinal SL | 1-Hydroxyethan-1,1-diphosphonsäure (ca. 58 - 61% Aktivsubstanzgehalt; INCI-Bezeichnung: Etidronic Acid, Aqua (Water)) (Solutia) |
| Texapon N28 | Laurylethersulfat-Natrium-Salz (mind. 26,5 % Aktivsubstanzgehalt; INCI-Bezeichnung: Sodium Laureth Sulfate) (Cognis) |
| Acrysol 22 | Acrylpolymer (ca. 29.5 - 30.5% Festkörper in Wasser; INCI-Bezeichnung: Acrylates/Steareth-20 Methacrylate Copolymer) |

### Vermischen mit der Entwicklerdispersion und Anwendung

Für den Färbeprozess wurde jeweils auf eine Strähne zu 80 % ergrauten Haares (Kerling) die 4-fache Menge der anwendungsbereiten Mischung appliziert. Nach einer Einwirkzeit von 30 Minuten bei 32 °C wurden die Strähnen ausgespült und mit einem üblichen Haarwaschmittel ausgewaschen. Die Färbung der Strähnen wurde nach dem Trocknen visuell unter der Tageslichtlampe beurteilt. Die Färbeergebnisse sind in der nachfolgenden Tabelle zusammengefasst.

**E1: N,N-Dimethyl-3-[(4-aminophenyl)amino]-N-{3-[(4-aminophenyl)amino]-propyl}-1-propanaminium-p-toluolsulfonat, Tetrahydrochlorid**

| Beispiel | Kupplerkomponente | erhaltene Nuance | Farbintensität |
|---|---|---|---|
| E1-1 | Resorcin | erdbraun | +++ |
| E1-2 | 3-Amino-2-methylamino-6-methoxypyridin | gewitterblau | +++ |
| E1-3 | 5-Amino-2-methylphenol | dunkelmagenta | +++ |
| E1-4 | 3-Am ino-2-hyd roxypyrid in | graubraun | +++ |
| E1-5 | 1,3-Bis(2,4-diaminophenoxy)propan | tintenblau | +++ |
| E1-6 | 2,7-Dihydroxynaphthalin | rauchbraun | ++ |

**E2: N-Ethyl-N-Methyl-3-[(4-aminophenyl)amino]-N-{3-[(4-aminophenyl)amino]-propyl}-1-propanaminium-p-toluolsulfonat, Tetrahydrochlorid**

| Beispiel | Kupplerkomponente | erhaltene Nuance | Farbintensität |
|---|---|---|---|
| E2-1 | Resorcin | braungrau | +++ |
| E2-2 | 3-Amino-2-methylamino-6-methoxypyridin | blaugrau | +++ |
| E2-3 | 5-Amino-2-methylphenol | aubergine | +++ |
| E2-4 | 3-Amino-2-hydroxypyridin | dunkelrubin | +++ |
| E2-5 | 1,3-Bis(2,4-diaminophenoxy)propan | schwarzblau | +++ |
| E2-6 | 2,7-Dihydroxynaphthalin | erdbraun | +++ |

**E3: N-Methyl-N-propyl-3-[(4-aminophenyl)amino]-N-{3-[(4-aminophenyl)amino]-propyl}-1-propanaminium-p-toluolsulfonat, Tetrahydrochlorid**

| Beispiel | Kupplerkomponente | erhaltene Nuance | Farbintensität |
|---|---|---|---|
| E3-1 | Resorcin | braungrau | +++ |
| E3-2 | 3-Amino-2-methylamino-6-methoxypyridin | blaugrau | +++ |
| E3-3 | 5-Amino-2-methylphenol | dunkelmagenta | +++ |
| E3-4 | 3-Amino-2-hydroxypyridin | graubraun | ++ |
| E3-5 | 1,3-Bis(2,4-diaminophenoxy)propan | schwarzblau | +++ |
| E3-6 | 2,7-Dihydroxynaphthalin | grüngrau | ++ |

**E4: N1,N4-Bis-{3-[(4-aminophenyl)amino]propyl}-N1-methylpiperazinium-p-toluolsulfonat, Hydrochloridsalz**

| Beispiel | Kupplerkomponente | erhaltene Nuance | Farbintensität |
|---|---|---|---|
| E4-1 | Resorcin | braungrau | ++ |
| E4-2 | 3-Amino-2-methylamino-6-methoxypyridin | blaugrau | +++ |
| E4-3 | 5-Amino-2-methylphenol | dunkelviolett | +++ |
| E4-4 | 3-Amino-2-hydroxypyridin | dunkelrubin | +++ |
| E4-5 | 1,3-Bis(2,4-diaminophenoxy)propan | schwarzblau | +++ |
| E4-6 | 2,7-Dihydroxynaphthalin | grünbraun (nutria) | ++ |

**E5: N,N-Dimethyl-3-[(4-amino-3-methylphenyl)amino]-N-{3-[(4-amino-3-methylphenyl)amino]propyl}-1-propanaminium-p-toluolsulfonat, Tetrahydrochlorid**

| Beispiel | Kupplerkomponente | erhaltene Nuance | Farbintensität |
|---|---|---|---|
| E5-1 | Resorcin | olivbraun | ++ |
| E5-2 | 3-Amino-2-methylamino-6-methoxypyridin | tannengrün | ++ |
| E5-3 | 5-Amino-2-methylphenol | mattviolett | ++ |
| E5-4 | 3-Amino-2-hydroxypyridin | dunkelbraun | ++ |
| E5-5 | 1,3-Bis(2,4-diaminophenoxy)propan | dunkelblau | ++ |
| E5-6 | 2,7-Dihydroxynaphthalin | oliv | + |

**E6: N-Ethyl-N-methyl-3-[(4-amino-3-methylphenyl)amino]-N-{3-[(4-amino-3-methylphenyl)amino]propyl}-1-propanaminium-p-toluolsulfonat, Tetrahydrochlorid**

| Beispiel | Kupplerkomponente | erhaltene Nuance | Farbintensität |
|---|---|---|---|
| E6-1 | Resorcin | oliv | ++ |
| E6-2 | 3-Amino-2-methylamino-6-methoxypyridin | nickelgrün | ++ |
| E6-3 | 5-Amino-2-methylphenol | dunkelviolett | ++ |
| E6-4 | 3-Amino-2-hydroxypyridin | graubraun | ++ |
| E6-5 | 1,3-Bis(2,4-diaminophenoxy)propan | dunkelblau | +++ |
| E6-6 | 2,7-Dihydroxynaphthalin | oliv | ++ |

**E7: N-Methyl-N-propyl-3-[(4-amino-3-methylphenyl)amino]-N-{3-[(4-amino-3-methylphenyl)amino]propyl}-1-propanaminium-p-toluolsulfonat, Tetrahydrochlorid**

| Beispiel | Kupplerkomponente | erhaltene Nuance | Farbintensität |
|---|---|---|---|
| E7-1 | Resorcin | olivbraun | + |
| E7-2 | 3-Amino-2-methylamino-6-methoxypyridin | nickelgrün | ++ |
| E7-3 | 5-Amino-2-methylphenol | aubergine | ++ |
| E7-4 | 3-Amino-2-hydroxypyridin | graubraun | ++ |
| E7-5 | 1,3-Bis(2,4-diaminophenoxy)propan | dunkelblau | +++ |
| E7-6 | 2,7-Dihydroxynaphthalin | oliv | ++ |

Farbintensität: +++ hoch ++ mittel + niedrig

## Patentansprüche

1. Mittel zur oxidativen Farbveränderung keratinischer Fasern, **dadurch gekennzeichnet, dass** es in einem kosmetischen Träger als Oxidationsfarbstoffvorprodukt vom Entwickler-Typ mindestens eine Verbindung der Formel (I) 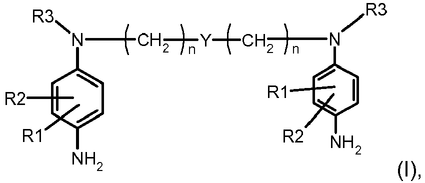 in der
R1 und R2 unabhängig voneinander für Wasserstoff, eine C₁-C₆-Alkylgruppe, eine C₂-C₆-Alkenylgruppe, eine C₁-C₆-Hydroxyalkylgruppe, eine C₂-C₆-Polyhydroxy-alkylgruppe, eine C₁-C₆-Alkoxygruppe oder ein Halogenatom stehen,
R3 für Wasserstoff, eine C₁-C₆-Alkylgruppe, eine C₂-C₆-Alkenylgruppe, eine C₂-C₆-Hydroxyalkylgruppe, eine C₂-C₆-Polyhydroxyalkylgruppe, eine C₁-C₆-Alkoxy-C₂-C₆-alkylgruppe, eine Cyano-C₁-C₆-alkylgruppe oder eine Aryl-C₁-C₆-alkylgruppe steht,
n für eine ganze Zahl von 2 bis 6 steht,
Y für eine kationische Gruppierung der Formeln (II) bis (V) steht, 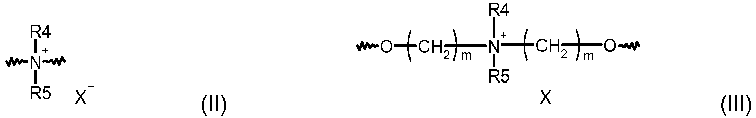 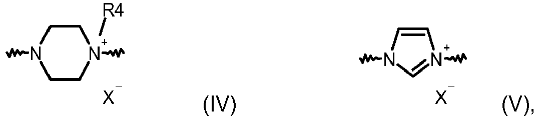
worin
R4, R5 unabhängig voneinander für eine C₁-C₆-Alkylgruppe, eine C₂-C₆-Alkenylgruppe, eine C₂-C₆-Hydroxyalkylgruppe, eine C₂-C₆-Polyhydroxyalkylgruppe, eine C₁-C₆-Alkoxy-C₂-C₆-alkylgruppe, eine Cyano-C₁-C₆-alkylgruppe oder eine Aryl-C₁-C₆-alkylgruppe stehen,
m für eine ganze Zahl von 2 bis 6 steht und
X⁻ für ein physiologisch verträgliches Anion steht,
und/oder dessen physiologisch verträgliches Salz enthält.

2. Mittel nach Anspruch 1, **dadurch gekennzeichnet, dass** es mindestens eine Verbindung der Formel (I) enthält, in der R1 und R2 unabhängig voneinander für Wasserstoff, eine C₁-C₆-Alkylgruppe, eine C₁-C₆-Alkoxygruppe oder ein Halogenatom stehen.

3. Mittel nach einem der Ansprüche 1 bis 2, **dadurch gekennzeichnet, dass** es eine Verbindung der Formel (I) enthält, in der R3 für Wasserstoff oder eine C₁-C₆-Alkylgruppe, insbesondere für Wasserstoff, steht.

4. Mittel nach einem der Ansprüche 1 bis 3, **dadurch gekennzeichnet, dass** es mindestens eine Verbindung der Formel (I) enthält, in der Y für eine kationische Gruppierung der Formeln (II) oder (IV) steht.

5. Mittel nach einem der Ansprüche 1 bis 3, **dadurch gekennzeichnet, dass** es mindestens eine Verbindung der Formel (I) enthält, in der R4 und R5 unabhängig voneinander für eine C₁-C₆-Alkylgruppe oder eine C₂-C₆-Alkenylgruppe stehen und/oder m bevorzugt für die Zahl 2 oder 3 steht.

6. Mittel nach einem der Ansprüche 1 bis 5, **dadurch gekennzeichnet, dass** die Verbindung gemäß Formel (I) ausgewählt wird aus der Gruppe, die gebildet wird aus Salzen des N,N-Dimethyl-3-[(4-amino-3-methylphenyl)amino]-N-{3-[(4-amino-3-methylphenyl)amino]propyl}-1-propanaminiums, des N-Ethyl-N-methyl-3-[(4-amino-3-methylphenyl)amino]-N-{3-[(4-amino-3-methylphenyl)amino]propyl}-1-propanaminiums, des N-Methyl-N-propyl-3-[(4-amino-3-methylphenyl)amino]-N-{3-[(4-amino-3-methylphenyl)amino]propyl}-1-propanaminiums, des N,N-Dimethyl-3-[(4-amino-2-methylphenyl)amino]-N-{3-[(4-amino-2-methyl-phenyl)amino]propyl}-1-propanaminiums, des N-Ethyl-N-methyl-3-[(4-amino-2-methylphenyl)amino]-N-{3-[(4-amino-2-methylphenyl)amino]propyl}-1-propanaminiums, des N-Methyl-N-propyl-3-[(4-amino-2-methylphenyl)amino]-N-{3-[(4-amino-2-methylphenyl)-amino]propyl}-1-propanaminiums, des N1,N4-Bis-{3-[(4-amino-3-methylphenyl)amino]-propyl}-N1-methylpiperaziniums, des N1,N4-Bis-{2-[(4-amino-3-methylphenyl)amino]ethyl}-N1-methylpiperaziniums, des N,N-Dimethyl-3-[(4-aminophenyl)amino]-N-{3-[(4-aminophenyl)amino]propyl}-1-propanaminiums, des N-Ethyl-N-methyl-3-[(4-aminophenyl)amino]-N-{3-[(4-aminophenyl)amino]propyl}-1-propanaminiums, des N-Methyl-N-propyl-3-[(4-aminophenyl)amino]-N-{3-[(4-aminophenyl)amino]-propyl}-1-propanaminiums, des N-Allyl-N-methyl-3-[(4-aminophenyl)amino]-N-{3-[(4-aminophenyl)amino]-propyl}-1-propanaminiums, des N1,N4-Bis-{3-[(4-aminophenyl)amino]propyl}-N1-methylpiperaziniums und/oder des N1,N4-Bis-{2-[(4-aminophenyl)amino]ethyl}-N1-methylpiperaziniums.

7. Mittel nach einem der Ansprüche 1 bis 6, **dadurch gekennzeichnet, dass** es die Verbindungen gemäß der Formel (I) in einem Gewichtsanteil von 0,001 bis 10,0 Gew.-%, insbesondere 0,05 bis 5 Gew.-%, bezogen auf Gesamtgewicht des anwendungsbereiten Mittels, enthält.

8. Mittel nach einem der Ansprüche 1 bis 7, **dadurch gekennzeichnet, dass** es zusätzlich mindestens ein Oxidationsfarbstoffvorprodukt vom Kuppler-Typ enthält, welches ausgewählt wird aus der Gruppe, die gebildet wird aus ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin und deren physiologisch verträglichen Salzen.

9. Verwendung eines Mittels nach einem der Ansprüche 1 bis 8 in oxidativen Färbemitteln für menschliche Haare zur Verbesserung der Grauabdeckung, der Egalisierung, der Farbintensität, der Haltbarkeit und/oder der Farbigkeit der Färbeergebnisse.

10. Verbindungen der Formel (I) nach einem der Ansprüche 1 bis 6.

## Claims

1. An agent for oxidatively changing the color of keratinic fibers, **characterized in that** it contains, in a cosmetic carrier, a compound of formula (I) as an oxidation dye precursor of the developer type 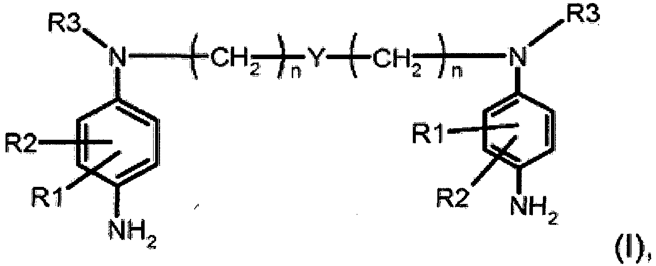 in which
R1 and R2, independently of one another, stand for hydrogen, a C₁-C₆ alkyl group, a C₂-C₆ alkenyl group, a C₁-C₆ hydroxyalkyl group, a C₂-C₆ polyhydroxyalkyl group, a C₁-C₆ alkoxy group or a halogen atom,
R3 stands for hydrogen, a C₁-C₆ alkyl group, a C₂-C₆ alkenyl group, a C₂-C₆ hydroxyalkyl group, a C₂-C₆ polyhydroxyalkyl group, a C₁-C₆-alkoxy-C₂-C₆-alkyl group, a cyano-C₁-C₆-alkyl group or an aryl-C₁-C₆-alkyl group,
n stands for an integer from 2 to 6,
Y stands for a cationic grouping of formulas (II) to (V), 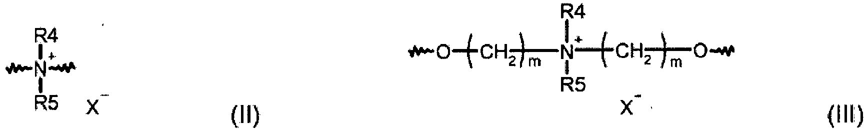 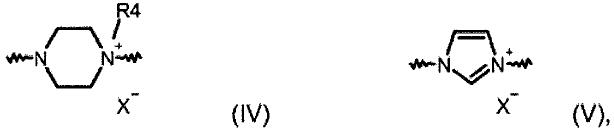 in which
R4 and R5, independently of one another, stand for a C₁-C₆ alkyl group, a C₂-C₆ alkenyl group, a C₂-C₆ hydroxyalkyl group, a C₂-C₆ polyhydroxyalkyl group, a C₁-C₆-alkoxy-C₂-C₆-alkyl group, a cyano-C₁-C₆-alkyl group or an aryl-C₁-C₆-alkyl group,
m stands for an integer from 2 to 6, and
X⁻ stands for a physiologically acceptable anion
and/or the physiologically acceptable salt thereof.

2. The agent according to claim 1, **characterized in that** it contains at least one compound of formula (I), in which R1 and R2, independently of one another, stand for hydrogen, a C₁-C₆ alkyl group, a C₁-C₆ alkoxy group or a halogen atom.

3. The agent according to either claim 1 or claim 2, **characterized in that** it contains a compound of formula (I), in which R3 stands for hydrogen or a C₁-C₆ alkyl group, in particular hydrogen.

4. The agent according to one of claims 1 to 3, **characterized in that** it contains at least one compound of formula (I), in which Y stands for a cationic grouping of formulas (II) or (IV).

5. The agent according to one of claims 1 to 3, **characterized in that** it contains at least one compound of formula (I), in which R4 and R5, independently of one another, stand for a C₁-C₆ alkyl group or a C₂-C₆ alkenyl group and/or m preferably stands for the number 2 or 3.

6. The agent according to one of claims 1 to 5, **characterized in that** the compound according to formula (I) is selected from the group comprising salts of N,N-dimethyl-3-[(4-amino-3-methylphenyl)amino]-N-{3-[(4-amino-3-methylphenyl)amino]propyl}-1-propanaminium, N-ethyl-N-methyl-3-[(4-amino-3-methylphenyl)amino]-N-{3-[(4-amino-3-methylphenyl)amino]propyl}-1-propanaminium, N-methyl-N-propyl-3-[(4-amino-3-methylphenyl)amino]-N-{3-[(4-amino-3-methylphenyl)amino]propyl}-1-propanaminium, N,N-dimethyl-3-[(4-amino-2-methylphenyl)amino]-N-{3-[(4-amino-2-methyl-phenyl)amino]propyl}-1-propanaminium, N-ethyl-N-methyl-3-[(4-amino-2-methyl-phenyl)amino]-N-{3-[(4-amino-2-methylphenyl)amino]propyl}-1-propanaminium, N-methyl-N-propyl-3-[(4-amino-2-methylphenyl)amino]-N-{3-[(4-amino-2-methylphenyl)-amino]propyl}-1-propanaminium, N1,N4-bis-{3-[(4-amino-3-methylphenyl)amino]-propyl}-N1-methylpiperazinium, N1,N4-bis-{2-[(4-amino-3-methylphenyl)amino]ethyl}-N1-methylpiperazinium, N,N-dimethyl-3-[(4-aminophenyl)amino]-N-{3-[(4-amino-phenyl)amino]propyl}-1-propanaminium, N-ethyl-N-methyl-3-[(4-aminophenyl)amino]-N-{3-[(4-aminophenyl)amino]propyl}-1-propanaminium, N-methyl-N-propyl-3-[(4-aminophenyl)amino]-N-{3-[(4-aminophenyl)amino]-propyl}-1-propanaminium, N-allyl-N-methyl-3-[(4-aminophenyl)amino]-N-{3-[(4-aminophenyl)amino]-propyl}-1-propanaminium, N1,N4-bis-{3-[(4-aminophenyl)amino]propyl}-N1-methylpiperazinium and/or N1,N4-bis-{2-[(4-aminophenyl)amino]ethyl}-N1-methylpiperazinium.

7. The agent according to one of claims 1 to 6, **characterized in that** it contains the compounds according to formula (I) in a proportion by weight of from 0.001 to 10.0 wt.%, in particular from 0.05 to 5 wt.%, based on the total weight of the ready-to-use agent.

8. The agent according to one of claims 1 to 7, **characterized in that** it further contains at least one oxidation dye precursor of the coupler type that is selected from the group comprising 3-aminophenol, 5-amino-2-methylphenol, 3-amino-2-chloro-6-methylphenol, 2-hydroxy-4-aminophenoxy-ethanol, 5-amino-4-chloro-2-methylphenol, 5-(2-hydroxyethyl)-amino-2-methylphenol, 2,4-dichloro-3-aminophenol, 2-aminophenol, 3-phenylenediamine, 2-(2,4-diaminophenoxy)ethanol, 1,3-bis(2,4-diaminophenoxy)propane, 1-methoxy-2-amino-4-(2-hydroxyethylamino)benzene, 1,3-bis(2,4-diaminophenyl)propane, 2,6-bis(2'-hydroxyethylamino)-1-methylbenzene, 2-({3-[(2-hydroxyethyl)amino]-4-methoxy-5-methylphenyl}amino)ethanol, 2-({3-[(2-hydroxyethyl)amino]-2-methoxy-5-methyl-phenyl}amino)ethanol, 2-({3-[(2-hydroxyethyl)amino]-4,5-dimethylphenyl}amino)ethanol, 2-[3-morpholin-4-ylphenyl)amino]ethanol, 3-amino-4-(2-methoxyethoxy)-5-methylphenylamine, 1-amino-3-bis-(2-hydroxyethyl)aminobenzene, resorcinol, 2-methylresorcinol, 4-chlororesorcinol, 1,2,4-trihydroxybenzene, 2-amino-3-hydroxypyridine, 3-amino-2-methylamino-6-methoxypyridine, 2,6-dihydroxy-3,4-dimethylpyridine, 3,5-diamino-2,6-dimethoxypyridine, 1-phenyl-3-methylpyrazol-5-one, 1-naphthol, 1,5-dihydroxynaphthalene, 2,7-dihydroxynaphthalene, 1,7-dihydroxynaphthalene, 1,8-dihydroxynaphthalene, 4-hydroxyindole, 6-hydroxyindole, 7-hydroxyindole, 4-hydroxyindoline, 6-hydroxyindoline, 7-hydroxyindoline and the physiologically acceptable salts thereof.

9. The use of an agent according to one of claims 1 to 8 in oxidative dyes for human hair for improving the coverage of gray hair, the levelling, the color intensity, the durability and/or the vividness of the color results.

10. The compounds of formula (I) according to one of claims 1 to 6.

## Revendications

1. Produit de coloration oxydante de fibres de kératine, **caractérisé en ce qu'**il contient comme précurseur de colorant d'oxydation de type développeur, dans un vecteur cosmétique, au moins un composé de formule (I) : 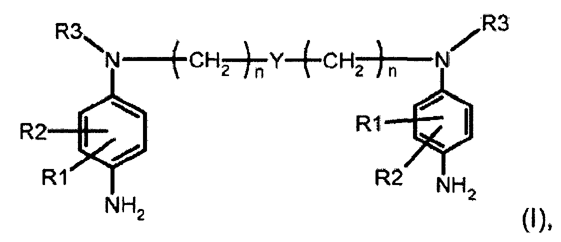 où
R1 et R2 indépendamment l'un de l'autre, représentent un hydrogène, un groupe C₁₋₆-alkyle, un groupe C₂₋₆-alkylène, un groupe C₁₋₆-hydroxyalkyle, un groupe poly-C₂₋₆-hydroxyalkyle, un groupe C₁₋₆-alcoxy ou un atome d'halogène,
R3 représente un hydrogène, un groupe C₁₋₆-alkyle, un groupe C₂₋₆-alkylène, un groupe C₁₋₆-hydroxyalkyle, un groupe poly-C₂₋₆-hydroxyalkyle, un groupe C₁₋₆-alcoxy-C₂₋₆-alkyle, un groupe cyano-C₁₋₆-alkyle ou un groupe aryl-C₁₋₆-alkyle,
n est un entier entre 2 et 6,
Y représente un groupe cationique de formule (II) à (V) : 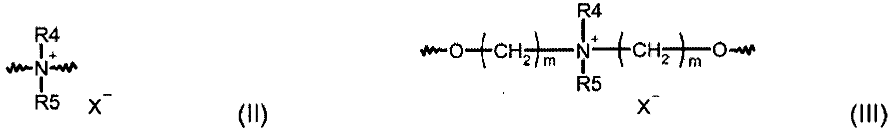 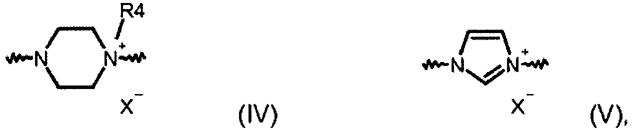 où
R4 et R5 indépendamment l'un de l'autre, représentent un groupe C₁₋₆-alkyle, un groupe C₂₋₆-alkylène, un groupe C₂₋₆-hydroxyalkyle, un groupe poly-C₂₋₆-hydroxyalkyle, un groupe C₁₋₆-alcoxy-C₂₋₆-alkyle, un groupe cyano-C₁₋₆-alkyle ou un groupe aryl-C₁₋₆-alkyle,
m est un entier entre 2 et 6,
X⁻ représente un anion physiologiquement compatible,
et/ou un de ses sels physiologiquement compatibles.

2. Produit selon la revendication 1, **caractérisé en ce qu'**il contient au moins un composé de formule (I) dans lequel R1 et R2 indépendamment l'un de l'autre, représentent un hydrogène, un groupe C₁₋₆-alkyle, un groupe C₁₋₆-alcoxy ou un atome d'halogène.

3. Produit selon une des revendications 1 ou 2, **caractérisé en ce qu'**il contient au moins un composé de formule (I) dans lequel R3 représente un hydrogène ou un groupe C₁₋₆ alkyle, en particulier un hydrogène.

4. Produit selon une des revendications 1 à 3, **caractérisé en ce qu'**il contient au moins un composé de formule (I) dans lequel Y représente un groupe cationique de formule (II) ou (IV).

5. Produit selon une des revendications 1 à 3, **caractérisé en ce qu'**il contient au moins un composé de formule (I) dans lequel R4 et R5 indépendamment l'un de l'autre, représentent un groupe C₁₋₆-alkyle ou un groupe C₂₋₆-alkylène, et/ou m est préférentiellement égal à 2 ou 3.

6. Produit selon une des revendications 1 à 5, **caractérisé en ce que** le composé de formule (I) est choisi dans le groupe constitué de sels de N,N-diméthyl-3-[(4-amino-3-méthylphényl)amino]-N-{3-[(4-amino-3-méthylphényl)amino]propyl}-1-propanaminium, N-éthyl-N-méthyl-3-[(4-amino-3-méthylphényl)amino]-N-{3-[(4-amino-3-méthylphényl)amino]propyl}-1-propanaminium, N-méthyl-N-propyl-3-[(4-amino-3-méthylphényl)amino]-N-{3-[(4-amino-3-méthylphényl)amino]propyl}-1-propanaminium, N,N-diméthyl-3-[(4-amino-2-méthylphényl)amino]-N-{3-[(4-amino-2-méthylphényl)amino]propyl}-1-propanaminium, N-éthyl-N-méthyl-3-[(4-amino-2-méthylphényl)amino]-N-{3-[(4-amino-2-méthylphényl)amino]propyl}-1-propanaminium, N-méthyl-N-propyl-3-[(4-amino-2-méthylphényl)amino]-N-{3-[(4-amino-2-méthylphényl)amino]propyl}-1-propanaminium, N1,N4-bis-{3-[(4-amino-3-méthylphényl)amino]propyl}-N1-méthylpipérazinium, N1,N4-bis-{2-[(4-amino-3-méthylphényl)amino]éthyl}-N1-méthylpipérazinium, N,N-diméthyl-3-[(4-aminophényl)amino]-N-{3-[(4-aminophényl)amino]propyl}-1-propanaminium N-éthyl-N-méthyl-3-[(4-aminophényl)amino]-N-{3-[(4-aminophényl)amino]propyl}-1-propanaminium, N-méthyl-N-propyl-3-[(4-aminophényl)amino]-N-{3-[(4-aminophényl)amino]propyl}-1-propanaminium, N-allyl-N-méthyl-3-[(4-aminophényl)amino]-N-{3-[(4-aminophényl)amino]propyl}-1-propanaminium, N1,N4-bis-{3-[(4-aminophényl)amino]propyl}-N1-méthylpipérazinium et/ou N1,N4-bis-{2-[(4-aminophényl)amino]propyl}-N1-méthylpipérazinium.

7. Produit selon une des revendications 1 à 6, **caractérisé en ce qu'**il contient les composés de formule (I) à hauteur de 0,001 à 10,0 % en poids, en particulier de 0,05 à 5 % en poids rapporté au poids total du produit prêt à l'emploi.

8. Produit selon une des revendications 1 à 7, **caractérisé en ce qu'**il contient en plus au moins un précurseur de colorant d'oxydation de type coupleur, choisi dans le groupe constitué de 3-aminophénol, 5-amino-2-méthylphénol, 3-amino-2-chloro-6-méthylphénol, 2-hydroxy-4-aminophénoxyéthanol, 5-amino-4-chloro-2-méthylphénol, 5-(2-hydroxyéthyl)amino-2-méthylphénol, 2,4-dichloro-3-aminophénol, 2-aminophénol, 3-phénylènediamine, 2-(2,4-diaminophénoxy)éthanol, 1,3-bis-(2,4-diaminophénoxy)propane, 1-méthoxy-2-amino-4-(2-hydroxyéthylamino)benzène, 1,3-bis-(2,4-diaminophényl)propane, 2,6-bis-(2'-hydroxyéthylamino)-1-méthylbenzène, 2-({3-[(2-hydroxyéthyl)amino]-4-méthoxy-5-méthylphényl}amino)éthanol, 2-({3-[(2-hydroxyéthyl)amino]-2-méthoxy-5-méthylphényl}amino)éthanol, 2-({3-[(2-hydroxyéthyl)amino]-4,5-diméthylphényl}amino)éthanol, 2-[3-morpholin-4-ylphényl)amino]éthanol, 3-amino-4-(2-méthoxyéthoxy)-5-méthylphénylamine, 1-amino-3-bis-(2-hydroxyéthyl)aminobenzène, résorcinol, 2-méthylrésorcinol, 4-chlororésorcinol, 1,2,4-trihydroxybenzène, 2-amino-3-hydroxypyridine, 3-amino-2-méthylamino-6-méthoxypyridine, 2,6-dihydroxy-3,4-diméthylpyridine, 3,5-diamino-2,6-diméthoxypyridine, 1-phényl-3-méthylpyrazol-5-one, 1-naphtol, 1,5-dihydroxynaphtaline, 2,7-dihydroxynaphtaline, 1,7-dihydroxynaphtaline, 1,8-dihydroxynaphtaline, 4-hydroxyindole, 6-hydroxyindole, 7-hydroxyindole, 4-hydroxyindoline, 6-hydroxyindoline, 7-hydroxyindoline, et leurs sels physiologiquement compatibles.

9. Utilisation d'un produit selon une des revendications 1 à 8 dans des produits de coloration par oxydation pour les cheveux humains pour améliorer la couverture des cheveux gris, l'égalisation, l'intensification de couleur, la durabilité et/ou la puissance de la coloration obtenue.

10. Composés de formule (I) selon une des revendications 1 à 6.
